(19) Europäisches Patentamt
European Patent Office
Office européen des brevets

(11) EP 2 653 095 B1

(12) **EUROPEAN PATENT SPECIFICATION**

(45) Date of publication and mention of the grant of the patent:
**20.12.2017 Bulletin 2017/51**

(51) Int Cl.:
***A61B 3/107*** *(2006.01)* ***A61F 2/16*** *(2006.01)*
***A61B 3/00*** *(2006.01)* *A61B 3/10* *(2006.01)*

(21) Application number: **13164413.0**

(22) Date of filing: **19.04.2013**

(54) **OPHTHALMIC MEASUREMENT PROGRAM AND OPHTHALMIC SIMULATION APPARATUS**

OPHTHALMISCHES MESSPROGRAMM UND OPHTHALMISCHE SIMULATIONSVORRICHTUNG

PROGRAMME DE MESURAGE OPHTHALMIQUE ET APPAREIL DE SIMULATION OPHTHALMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.04.2012 JP 2012097179**
**31.08.2012 JP 2012190953**

(43) Date of publication of application:
**23.10.2013 Bulletin 2013/43**

(73) Proprietor: **Nidek Co., Ltd.**
**Aichi 433-0038 (JP)**

(72) Inventor: **BAN, Yukinobu**
**Gamagori-shi, Aichi 443-0038 (JP)**

(74) Representative: **Winter, Brandl, Fürniss, Hübner, Röss, Kaiser, Polte - Partnerschaft mbB**
**Patent- und Rechtsanwaltskanzlei**
**Alois-Steinecker-Strasse 22**
**85354 Freising (DE)**

(56) References cited:
**DE-A1-102009 008 876**
**JP-A- 2010 200 915**
**US-A1- 2002 186 346**
**US-A1- 2007 052 927**
**US-A1- 2009 281 552**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 653 095 B1

**Description**

BACKGROUND OF THE INVENTION

1. Field of the Invention

The present invention relates to an ophthalmic measurement program for carrying out a simulation of a retinal image of an examinee's eye at a time when an intraocular lens is implanted in the eye, and an ophthalmic simulation apparatus for carrying out the same.

2. Description of Related Art

Conventionally, an intraocular lens that replaces a crystalline lens of an examinee's eye is implanted into the eye in cataract surgery. When the diopter of the intraocular lens is determined, the corneal curvature and the ocular axial length of the eye are measured in advance. Then, by substituting a result of the measurement in a predetermined formula for obtaining a diopter of an intraocular lens, the diopter of the intraocular lens is found.

However, although the diopter that is found in the predetermined formula for obtaining a diopter of an intraocular lens is an ideal diopter for the examinee, it could happen that an intraocular lens with the found diopter does not exist. For example, because intraocular lenses are prepared in 0.5 D steps, a slight refractive error could exist in the intraocular lens depending on the found diopter.

In addition, an apparatus for obtaining corneal wavefront aberration of an examinee's eye and carrying out a seeing simulation of how the eye into which an intraocular lens is implanted sees a target image with the use of software is proposed (see US 2009/281552 A1 and Japanese Unexamined
Patent Application Publication No. 2009-34451).

It is to be noted that usually only the manufacturers of the intraocular lenses know detailed design values of the intraocular lenses. In order that a person other than the manufacturers can obtain the exact detailed design values of the intraocular lenses, it is necessary for the person to obtain the lens shapes, and the image-formation states of the intraocular lenses, which is not realistic. Under this circumstance, a favorable simulation is desired.

SUMMARY OF THE INVENTION

In view of the above problems in the conventional art, it is an object of the present invention to provide an ophthalmic measurement program that is capable of carrying out a favorable simulation relating to implantation of an intraocular lens, and an ophthalmic simulation apparatus that is capable of carrying out the same.

To achieve the object and in accordance with the purpose of the present invention, an ophthalmic measurement program according to claim 1 and an ophthalmic simulation apparatus according to claim 13 are provided. Additional objects and advantages of the invention are set forth in the description which follows.

BRIEF DESCRIPTION OF THE DRAWINGS

The accompanying drawings illustrate embodiments of the present invention and, together with the description, serve to explain the objects, advantages and principles of the invention. In the drawings,

FIG. 1 is a block diagram for illustrating the entire configuration of a simulation apparatus (ophthalmic measurement apparatus) including an ophthalmic measurement program according to the present embodiment of the present invention;
FIG. 2 is a flow chart showing one example of steps of a simulation according to the present embodiment of the present invention;
FIG. 3 is a view for illustrating a CT·REF measurement screen in the operations of an intraocular lens selection apparatus according to the present embodiment of the present invention;
FIG. 4 is a view for illustrating a screening screen in the operations of the intraocular lens selection apparatus according to the present embodiment of the present invention;
FIG. 5 is a view for illustrating an ocular axial length measurement screen in the operations of the intraocular lens selection apparatus according to the present embodiment of the present invention;
FIG. 6 is a view for illustrating a data input screen in the operations of the intraocular lens selection apparatus according to the present embodiment of the present invention;
FIG. 7 is a view for illustrating an IOL selection screen in the operations of the intraocular lens selection apparatus according to the present embodiment of the present invention;

FIG. 8 is a view for illustrating a seeing simulation screen in the operations of the intraocular lens selection apparatus according to the present embodiment of the present invention;

FIG. 9 is an IOL formula in SRT formula that defines one example of an IOL formula;

FIG. 10 is a flow chart for illustrating a method for a seeing simulation according to the present embodiment of the present invention; and

FIG. 11 is a view showing an optical relation.

DETAILED DESCRIPTION OF THE EMBODIMENTS

A detailed description of one embodiment for carrying out a program according to the present invention with the use of a computer is provided below with reference to the accompanying drawings. FIG. 1 to FIG. 11 are views relating to the present embodiment of the present invention.

<SUMMARY>

An ophthalmic measurement program according to present embodiment of the present invention is arranged to make a computer carry out at least any one of an aberration data obtaining step, a post-operative calculation step, an image creating step, and a display step.

The aberration data obtaining step is arranged to obtain data on corneal wavefront aberration of a cornea of an examinee's eye that is measured with the use of a corneal shape measurement apparatus 10. It is also preferable that the present step should obtain the corneal wavefront aberration data that is transferred to the computer after the corneal wavefront aberration data is calculated by being subjected to arithmetic processing by the corneal shape measurement apparatus 10. It is also preferable that the present step should obtain the corneal wavefront aberration data by making the computer calculate the corneal wavefront aberration data based on a measurement result that is obtained by the corneal shape measurement apparatus 10.

The post-operative calculation step is arranged to calculate a post-operative sphere power, a post-operative astigmatic power, and a post-operative astigmatic axial angle of the entire eye based on an estimated post-operative refractive power of the examinee's eye that is calculated by a predetermined intraocular lens formula arranged to obtain a post-operative refractive power (ERROR). For example, an estimated post-operative spherical equivalent refractive power and an estimated post-operative spherical refractive power are considered as the estimated post-operative refractive power. It is preferable that the display step should be arranged to display the calculated post-operative sphere power, post-operative astigmatic power, and post-operative astigmatic axial angle of the entire eye as a post-operative refractive error on a monitor. It is preferable that the estimated post-operative refractive power should be calculated in diopter. It is more preferable that a predetermined formula arranged to calculate the post-operative sphere power, the post-operative astigmatic power, and the post-operative astigmatic axial angle of the entire eye should be used. Examples of the predetermined formula include a formula arranged to calculate the post-operative sphere power, the post-operative astigmatic power, and the post-operative astigmatic axial angle of the entire eye based a combined refractive power of a corneal refractive power of the examinee's eye and a refractive power of an intraocular lens to be implanted into the examinee's eye, and an ocular axial length of the examinee's eye. For example, the following procedure is considered as the post-operative calculation step.

For example, if a toric intraocular lens is used as the intraocular lens, the post-operative calculation step calculates the post-operative sphere power, the post-operative astigmatic power, and the post-operative astigmatic axial angle of the entire eye assuming that the toric intraocular lens is disposed so as to cancel corneal astigmatism.

For example, the post-operative calculation step combines the corneal refractive power of the examinee's eye and a refractive power of the intraocular lens that is apart from the cornea into the eye by a depth ACD of an estimated post-operative anterior chamber, and calculates the post-operative sphere power, the post-operative astigmatic power, and the post-operative astigmatic axial angle of the entire eye with respect to a retina that is located at a position corresponding to the ocular axial length of the examinee's eye.

For example, the post-operative calculation step varies the depth ACD of the estimated post-operative anterior chamber included in a predetermined formula. Then, the post-operative calculation step calculates a depth pACD of an estimated post-operative anterior chamber at which the estimated post-operative refractive power that is calculated by the predetermined intraocular lens formula corresponds to the post-operative spherical power of the entire eye. Then, the post-operative calculation step calculates the post-operative sphere power, the post-operative astigmatic power, and the post-operative astigmatic axial angle of the entire eye by using the obtained depth pACD of the estimated post-operative anterior chamber and the predetermined formula. It is also preferable that the display step should display on the monitor the depth pACD of the estimated post-operative anterior chamber at which the estimated post-operative refractive power corresponds to the post-operative spherical power.

For example, the post-operative calculation step varies the depth ACD of the estimated post-operative anterior chamber included in a predetermined formula. Then, the post-operative calculation step calculates a depth pACD of an estimated post-operative anterior chamber at which an estimated post-operative spherical equivalent refractive power that is calculated by the predetermined intraocular lens formula corresponds to a post-operative spherical equivalent power of the entire eye. Then, the post-operative calculation step calculates the post-operative sphere power, the post-operative astigmatic power, and the post-operative astigmatic axial angle of the entire eye by using the obtained depth pACD of the estimated post-operative anterior chamber. It is also preferable that the display step should display on the monitor the depth ACD of the estimated post-operative anterior chamber at which the estimated post-operative spherical equivalent refractive power corresponds to the post-operative spherical equivalent power of the entire eye.

For example, in the post-operative calculation step, the post-operative refractive power of the entire eye is calculated based on the combined refractive power of the corneal refractive power of the examinee's eye and the refractive power of the intraocular lens to be implanted into the examinee's eye, and the ocular axial length of the examinee's eye. Then, the post-operative calculation step calculates the post-operative sphere power, the post-operative astigmatic power, and the post-operative astigmatic axial angle of the entire eye under a condition that the calculated post-operative refractive power of the entire eye corresponds to the estimated post-operative refractive power of the examinee's eye that is calculated by the predetermined intraocular lens formula.

In the above description, concerning the spherical equivalent power of the entire eye that is used when calculating the depth of the estimated post-operative anterior chamber, the refractive power of the intraocular lens is determined based on the sphere power, the astigmatic power, and the ocular axial length. Alternatively, the refractive power of the intraocular lens is determined based on only the sphere power. That is, the astigmatic power, and the ocular axial length are regarded as zero. In this manner, a depth of an estimated post-operative anterior chamber that is more in accordance with the intraocular lens formula can be be calculated.

Examples of the predetermined intraocular lens formula include SRK formula, SRK II formula, BINKHORST formula, HOFFER-Q formula, HOLLADAY formula, and HAIGIS formula.

The image creating step calculates a coefficient of a polynomial that includes the post-operative sphere power, the post-operative astigmatic power, and the post-operative astigmatic axial angle that are calculated in the post-operative calculation step, and high-order aberration of the corneal wavefront aberration that is obtained in the aberration data obtaining step. The polynomial defines a formula that is arranged to approximate the wavefront aberration data of the entire eye. The image creating step performs an inverse operation on the wavefront aberration data with the use of the calculated coefficient of the polynomial, and creates a simulation image based on the wavefront aberration data on which the inverse operation is performed. Further, it is also preferable that the image creating step should calculates a coefficient of a polynomial that includes a spherical aberration amount of the intraocular lens, the polynomial being arranged to approximate the wavefront aberration data of the entire eye.

It is also preferable that the program should further include the step of calculating the estimated post-operative refractive power of the examinee's eye by substituting parameters relating to a corneal shape of the examinee's eye, an ocular axial length of the examinee's eye, and the intraocular lens to be implanted into the examinee's eye in the predetermined intraocular lens formula. Examples of the parameters relating to the corneal shape of the examinee's eye include a corneal refractive power, a corneal radius of curvatures, and a corneal thickness. It is also preferable that not only the data on the corneal shape but also data on a rear surface of the cornea should be used. Examples of the parameters relating to the intraocular lens include a refractive power of the intraocular lens to be implanted into the eye, an A constant, and a surgeon factor.

Hereinafter, a description of the present embodiment of the present invention will be provided below with reference to the accompanying drawings. FIG. 1 is a block diagram for illustrating the entire configuration of a simulation apparatus (ophthalmic measurement apparatus) including an ophthalmic measurement program according to the present embodiment of the present invention. FIG. 2 is a flow chart showing one example of steps of a simulation according to the present embodiment of the present invention.

A simulation apparatus 1 is mainly used to carry out a simulation of a retinal image of an examinee's eye (patient's eye) at the time when an intraocular lens is implanted in the eye. The simulation apparatus 1 includes a CPU (arithmetic processing unit) 30, a memory 35, an operating input unit (hereinafter, referred to as the input unit) 40, a printer 43, a monitor 50, an image processing unit 31, and other constituent elements that are connected via buses.

The CPU 30 is arranged to control an operation of each unit in accordance with a retinal image simulation program, and control programs of various kinds. The input unit 40 defines an input device that is operated by an examiner. A pointing device such as a switch, a keyboard, a mouse, and a touch panel can be used as the input unit 40. The image processing unit 31 is arranged to control a display screen of a monitor 50 that is arranged to display a variety of data, and simulation images. The memory 35 defines a memory unit, and is arranged to store a variety of programs that are executed in the CPU 30 (e.g., a variety of control programs for apparatus operation, and programs for retinal image simulations), a variety of intraocular lens (IOL) information, surgeon information, measurer information, a variety of formulae for calculating IOL powers (SRK-T formula, SRK formula, HOLLADAY formula and other formulae), and other information. The memory 35 is used as a memory device. A semiconductor memory, magnetic-storage device, and an optical recorder are preferably used as the memory 35. The monitor 50 is used as an output device, and controlled by the CPU 30. The monitor 50 according to the present embodiment of the present invention defines a touch panel with which the examiner is capable of performing input operation, and functions also as at least a part of the input unit 40. The printer 43 is arranged to print results of the simulations.

It is also preferable that a commercially available personal computer, in which the retinal image simulation program is installed, should be used as the CPU 30, the input unit 40, the memory 35, the monitor 50, and the image processing unit 31.

A corneal shape measurement apparatus 10 and an ocular axial length measurement apparatus 20 that are arranged to obtain measurement data of the examinee's eye that are used in selecting the intraocular lens are connected to the simulation apparatus 1. The simulation apparatus 1, and the corneal shape measurement apparatus 10 and the ocular axial length measurement apparatus 20 may be of a multi-component assembled construction. Alternatively, the simulation apparatus 1, and at least one of the corneal shape measurement apparatus 10 and the ocular axial length measurement apparatus 20 may be of a monolithic construction.

The corneal shape measurement apparatus 10 is arranged to measure a corneal shape of the examinee's eye by projecting measurement light onto a cornea of the examinee's eye and photo-receiving the light reflected from the cornea. Data on the corneal shape that is measured by the corneal shape measurement apparatus 10 is analyzed, whereby data that is necessary for a simulation for implanting the intraocular lens into the eye such as a corneal refractive power and corneal wavefront aberration of the examinee's eye is obtained.

It is also preferable that the corneal refractive power and the corneal wavefront aberration should be obtained with the use of an arithmetic control unit provided to the corneal shape measurement apparatus 10, or should be obtained with the use of an arithmetic processing unit provided to the simulation apparatus.

Examples of the corneal shape measurement apparatus 10 include an apparatus arranged to measure a corneal shape of an examinee's eye by projecting a placido ring target onto a cornea of the examinee's eye and photo-receiving reflection light of the placido ring target, and an apparatus arranged to measure a corneal shape of an examinee's by using a principle of optical interference (i.e., an anterior-segment optical coherence tomography).

The corneal shape measurement apparatus 10 according to the present embodiment of the present invention includes a placido plate 11, an illumination light source 12, an anterior-segment image-pickup optical system 15, and a control unit 16. The placido plate 11 includes a plurality of placido rings. The illumination light source 12 is arranged to almost uniformly illuminate a ring pattern of the placido plate 11. The anterior-segment image-pickup optical system 15 includes a photographing lens 13 and a two-dimensional image-pickup element 14, and is arranged to photograph an image of the ring pattern projected onto the cornea of the examinee's eye. The anterior-segment image-pickup optical system 15 is arranged to photograph a placido image. It is also preferable that the anterior-segment image-pickup optical system 15 should be also arranged to photograph an image of an anterior segment of the examinee's eye that includes a pupil portion of the examinee's eye. Further, it is also preferable that the anterior-segment image-pickup optical system 15 should be also arranged to detect a bright point on a corneal vertex formed on the cornea of the examinee's eye with the use of a predetermined projection optical system (not illustrated).

The corneal shape measurement apparatus 10 according to the present embodiment of the present invention includes a measurement optical system arranged to measure eye refractive power distribution or wavefront aberration of the examinee's eye by projecting a measurement light bundle onto a fundus of the examinee's eye and photo-receiving the light bundle reflected from the fundus (e.g., a phase-difference method disclosed in Japanese Unexamined Patent Application Publication No. Hei10-108837, and an ophthalmic aberration indicator using a Shack-Hartmann sensor) (not illustrated). The simulation apparatuses 1 and the corneal shape measurement apparatus 10 are connected to each other preferably via LAN, and the variety of data (e.g., the data on the corneal wavefront aberration of the examinee's eye) obtained by the corneal shape measurement apparatus 10 are transferred to the memory unit 35 that functions as a database.

In addition, examples of the ocular axial length measurement apparatus 20 include an ultrasonic ocular axial length measurement apparatus, and an optical interferometric ocular axial length measurement apparatus. The ultrasonic ocular axial length measurement apparatus is arranged to measure an ocular axial length of an examinee's eye by receiving a return echo from the examinee's eye with the use of an ultrasonic probe. The optical interferometric ocular axial length measurement apparatus is arranged to optically measure an ocular axial length of an examinee's eye by using a principle of optical interference.

In addition, the IOL information stored in the memory unit 35 defines information on a variety of models of intraocular lenses provided by a variety of manufacturers, and preferably includes a model name, a manufacturer name, an A constant, and an post-operative ACD: an estimated depth of an anterior chamber (mm) for each model. It is to be noted that spherical aberration SA (correction spherical aberration) of each intraocular lens disclosed in the present embodiment of the present invention defines spherical aberration to be corrected when a predetermined intraocular lens is implanted into the examinee's eye, and is used together with spherical aberration of the cornea of the examinee's eye to calculate post-operative spherical aberration of the examinee's eye as an estimated value.

It is to be noted that when the manufactures obtain the spherical aberration SA of the intraocular lenses, corneal spherical aberration is calculated based on a corneal shape of a predetermined model eye of which design conditions are already known (e.g., +0.30 μm), and at the same time, spherical aberration of the entire model eye is calculated based on wavefront aberration of the entire model eye into which the intraocular lens is implanted (e.g., +0.10 μm), and then corrected spherical aberration is calculated by subtracting the corneal spherical aberration from the spherical aberration of the entire model eye, whereby the spherical aberration SA is obtained (+0.10-(+0.30) = -0.20). The above-described arithmetic processing can be carried out with the use of optical simulation software that is capable of making a simulation of wavefront aberration of a predetermined model eye into which an intraocular lens having predetermined optical characteristics is implanted. In addition, while the above description was made assuming that the spherical aberration SA is provided by the manufactures, the spherical aberration SA can be calculated also by obtaining optical characteristics (a lens radius of curvatures, a refractive index, a lens thickness, a conic constant, and an aspherical constant) of an intraocular lens that are either provided by the manufactures or obtained with the use of a variety of measurement devices, and then carrying out a simulation of wavefront aberration of a predetermined model eye into which an intraocular lens is implanted.

The surgeon information stored in the memory unit 35 defines information on a surgeon who performs intraocular lens implantation surgery, and preferably includes a name of the surgeon, IOL information on an intraocular lens the surgeon uses, target post-operative residual spherical aberration of the examinee's eye desired by the surgeon. In the surgeon information, the A constant, the post-operative ACD (mm), and the SF (SURGERY FACTOR) among the IOL information registered for each surgeon can be changed for each surgeon.

The measurer information stored in the memory unit 35 defines information on a measurer who measures an ocular axial length of an examinee's eye with the use of an ultrasonic diagnosing apparatus, and preferably includes a name of the measurer, and a correction value corresponding to the measurer's name. The IOL information, the surgeon information, and the measurer information can be changed such that new information is added to be registered or such that the registered information is changed on the respective setting and registration screens.

FIG. 2 is a flow chart showing one example of steps of an intraocular lens selection apparatus according to the present embodiment of the present invention. As shown in the flow chart, modes for making selection of an intraocular lens (navigation modes) are provided, which include a CT·REF measurement mode, a screening mode, an ocular axial length measurement mode, a data input mode, an IOL selection mode, a seeing comparison mode, and a print mode. In addition to these modes, a variety of setting modes, a reprint mode, and a detail confirmation mode are provided. An area 70 arranged to display a mode list indicating the modes in order, and the mode presently set is provided as shown in FIG. 3 to FIG. 6. The mode presently set is filled with a color that is thinner than the other modes displayed.

In addition, when changing the mode, a mode shift button 74 provided on the monitor 50, with which the mode is shifted to a next mode, and a mode shift button 75 on the monitor 50, with which the mode is shifted to a previous mode, are used as shown in FIG. 3 to FIG. 6.

When the program is activated and the navigation mode is selected, the CPU 30 shifts the mode to a CT·REF measurement screen 80 as shown in FIG. 3. The CT·REF measurement screen 80 includes an area 81 arranged to display patient information, an area 82 arranged to explain a procedure of the measurement, and a measurement data capture button 83, and a patient selection button 84. The measurement data capture button 83 is arranged to obtain the data preferably via a USB memory when the corneal shape measurement apparatus 10 is not connected to the simulation apparatus 1 via LAN.

At this stage, when the patient selection button 84 is clicked on with the use of a mouse 41, a previously-registered patient list is displayed on the monitor 50 (not illustrated) . By selecting a patient who is concerned from the list, patient information (e.g., an ID number, a name, an age, an eye to be measured, a sex, and an examination date) is obtained. It is also preferable that the patient information should be input with the use of a keyboard 42 and the mouse 41.

Next, following the explanation displayed in the area 82, the examiner measures eye refractive power distribution or wavefront aberration of the examinee's eye (i.e., performs REF measurement) with the use of the corneal shape measurement apparatus 10. Then, the examiner photographs a placido ring image and an anterior segment image of the examinee's eye (i.e., performs CT measurement) with the use of the corneal shape measurement apparatus 10. At this stage, the control unit 16 of the corneal shape measurement apparatus 10 obtains detailed information (i.e., measurement data) relating to the cornea of the examinee's eye such as corneal curvature distribution, a three-dimensional shape of a corneal surface of the examinee's eye (corneal height distribution of the examinee's eye in a measurement optical axis L1), and corneal refractive power distribution based on the obtained placido ring image. In addition, the control unit 16 compares the obtained corneal height distribution with corneal height distribution in which aberration is assumed to be zero, and based on information on the subtraction, calculates wavefront aberration of the cornea of the examinee's eye in a predetermined analytical diameter. It is to be noted that when a portion of the placido ring image is obstructed by an eyelid of the examinee's eye, the analytical diameter could become smaller. In addition, the control unit 16 extracts a corneal vertex position and a pupillary center position of the examinee's eye based on the photographed anterior segment image (an image including the bright point on the corneal vertex and the pupil portion), and obtains an amount and a direction of the deviation between the corneal vertex position and the pupillary center position through arithmetic processing.

When the arithmetic processing by the control unit 16 is completed as described above, the obtained measurement data is automatically transferred to the memory unit 35. In this case, it is also preferable that the measurement data stored in the memory unite 35 should be checked on the monitor 50. While the calculation of the measurement data is carried out with the use of the control unit 16 of the corneal shape measurement apparatus 10 in the above description, it is also preferable that the arithmetic processing is carried out by the CPU 30.

After the CT·REF measurement is completed, and the mode shift button 74 is clicked on by the examiner operating the mouse 41, the CPU 30 shifts the screen to a screening screen 100 as shown in FIG. 4.

The screening screen 100 includes an area 101 arranged to display information on the anterior segment of the examinee's eye based on the measurement data transferred from the corneal shape measurement apparatus 10. In addition, the screening screen 100 includes an area 102 arranged to display information to be input in order to carry out a power calculation of the IOL, and an area 103 arranged to display a measurement result (e.g., an eye refractive power value SCA, VD, and corneal refractive powers K1 and K2) that is obtained in the CT·REF measurement mode (as shown also in FIG. 5 to FIG.8).

The area 101 is arranged to display a result of screening, a diagnostic comment, MDist, corneal spherical aberration, corneal high-order aberration, and an analytical diameter of corneal spherical aberration. Among them, the result of the screening defines an analytical result obtained by the CPU 30 based on the presence/absence of corneal abnormalities in the examinee's eye based on the corneal curvature distribution of the examinee's eye that is obtained as described above, and the result is presumed among a normal eye (an astigmatic amount equal to or less than 0.5 D), an astigmatic eye with an astigmatic amount more than 0.5D, keratoconus suspected, keratoconus, corneal degeneration, a corneal transplantation performed eye, a myopic correction performed eye, a hyperopic correction performed eye, and the other. The diagnostic comment defines a comment that the CPU 30 displays based on the result of the screening. In FIG. 4, it is indicates that the percentage that the examinee's eye is a normal eye (NRM) is 99% while the percentage that the examinee's eye is the other (OTHER) kind of eye is 1%. It is to be noted that a detail of the method for the corneal analysis described above is disclosed in Japanese Unexamined Patent Application Publication No. 2005-288176).

In addition, the MDist indicates the amount (eccentric distance) and the direction of the deviation between the corneal vertex and the pupillary center of the examinee's eye that are obtained as described above, and the amount of the deviation is expressed in the unit mm and the direction of the deviation is expressed in the unit degree. In addition, the CPU 30 makes a determination as to which the examinee's eye corresponds to among a normal eye (NORMAL), a suspect eye (SUSPECT), and an abnormal eye (ABNORMAL) in accordance with the deviation amount. In the present embodiment of the present invention, the examinee's eye is determined to be a normal eye when the previously-obtained eccentric distance MDist falls within the range from equal to or more than 0 mm to less than 0.3 mm, determined to be a suspect eye when the previously-obtained eccentric distance MDist falls within the range from equal to or more than 0.3 mm to less than 0.5 mm, or determined to be an abnormal eye when the previously-obtained eccentric distance MDist falls within the range equal to or more than 0.5 mm. Then, when the examinee's eye is determined to be a normal eye, the area indicating the deviation amount is filled with a green color. When the examinee's eye is determined to be a suspect eye, the area indicating the deviation amount is filled with a yellow color. When the examinee's eye is determined to be an abnormal eye, the area indicating the deviation amount is filled with a red color.

In addition, the corneal spherical aberration indicates spherical aberration that is one of high-order aberration components in the cornea of the examinee's eye, and an amount of the aberration is displayed in the unit $\mu$m, and an analytical diameter of the corneal spherical aberration is also displayed. In this case, the CPU 30 calculates the corneal spherical aberration of the examinee's eye by picking up a component of the spherical aberration of the examinee's eye by using the Zernike's polynomial based on a component of the wavefront aberration of the cornea of the examinee's eye that is obtained as described above.

In addition, the corneal high-order aberration indicates the sum of the high-order aberration of the examinee's eye (the high-order aberration defines a component of the Zernike's polynomial that is equal to or more than a third-order component), and an amount of the aberration is displayed in the unit $\mu$m, and an analytical diameter of the aberration is also displayed. In this case, the CPU 30 calculates the corneal high-order aberration of the examinee's eye by picking up a component of the high-order aberration of the examinee's eye by using the Zernike's polynomial (the sum of components of the Zernike's polynomial that are equal to or more than third-order components) based on a component of the wavefront aberration of the cornea of the examinee's eye that is obtained as described above.

In addition, the analytical diameter of the corneal spherical aberration indicates a corneal region of the examinee' s eye that is obtained at the time when the control unit 16 of the corneal shape measurement apparatus 10 obtains the wavefront aberration of the examinee's eye. Concerning the analytical diameter, analysis in the region of 6 mm in diameter is made as a standard because a general optical part of an intraocular lens has a diameter of 6 mm.

At this stage, the CPU 30 makes a determination as to whether the analytical diameter used in the analysis is normal or abnormal. The analytical diameter is determined to be normal when the analytical diameter is equal to 6 mm, abnormality suspected when the analytical diameter is from 5.0 mm to less than 6 mm, and abnormal when the analytical diameter is equal to or less than 5.0 mm. When the analytical diameter is determined to be normal, the area indicating the corneal diameter is filled with a green color. When the examinee's eye is determined to be abnormality suspected, the area indicating the corneal diameter is filled with a yellow color. When the examinee's eye is determined to be abnormal, the area indicating the corneal diameter is filled with a red color.

Hereinafter, a description of the area 102 will be provided. In the area 102, the IOL power formula to be selected, a history of surgery on the cornea, a surgeon name, a presence/absence of an experience of the intraocular lens implantation, VD: a distance between corneal vertexes, and a corneal curvature to be used. When a pull-down button that is provided in a region where the name of the IOL power formula is displayed is clicked with the mouse 41, a list of the formulae stored in the memory unit 35 is displayed, and clicking on a desired formula allows the desired formula to be selected. In addition, a formula that is used previous time is set to be default (stored), so that the formula that is used previous time is used without change in the following selection. Examples of the IOL power formula include Camellin-Calossi formula, SRK formula, SRK-2 formula, SRK-T formula, BINKHORST formula, HOFFER-Q formula, and Holladay formula.

In addition, when a pull-down button that is provided in a region where the history of surgery is displayed is clicked with the mouse 41, a list of histories of the surgeries is displayed, and clicking on a history of surgery that concerns allows the history of surgery to be selected. In the present embodiment of the present invention, the history of surgery on the cornea can be selected from no surgery experienced (default), incision experienced (RK, AK, CK), laser ablation experienced (PRK, LASIK), implantation experienced, and PTK experienced. In addition, when any one of incision experienced (RK, AK, CK), laser ablation experienced (PRK, LASIK), implantation experienced, and PTK experienced is selected in selecting the history of surgery, a correction value of the selected surgery is displayed, and the correction value is used for power calculation of the intraocular lens in the case of presence of the history of corneal surgery.

In addition, when a pull-down button that is provided in a region where the surgeon name is displayed is clicked on with the mouse 41, a list of surgeon names is displayed, and clicking on a surgeon name that concerns allows the surgeon to be selected.

In addition, when a pull-down button that is provided in a region where the presence/absence of the experience of the intraocular lens implantation is displayed is clicked on with the mouse 41, two options consisting of first time, and second or more time are displayed, and clicking on an option that concerns allows the option to be selected. This information is used as a variable of Camellin-Calossi formula.

In addition, when a pull-down button that is provided in a region where the VD is displayed is clicked on with the mouse 41, a list of selectable VDs is displayed, and clicking on a VD that concerns allows the VD to be selected. In the present embodiment of the present invention, the VD can be selected among VDs of 10.50 mm, 12.00 mm, 13.75 mm, 15.00 mm, and 16.50 mm. This information is used as a variable of Camellin-Calossi formula.

In addition, when any one of three selectable regions (radio buttons) that are provided in order to specify the corneal curvature to be used in the intraocular lens power calculation is clicked on (selected) with the mouse 41, any one of "average power in pupil", "average value of K1, K2", and "value of K2" is selected as the corneal curvature to be used in the intraocular lens power calculation. In addition, the CPU 30 make a determination as to which value is a recommended corneal curvature value based on a corneal refractive power that is calculated from the corneal curvature of the examinee's eye that is stored in the memory unit 35. In addition, the CPU 30 makes a determination (selects) as to whether a value of corneal radius of curvatures in a region 3 mm in diameter based on a corneal center (corneal vertex) should be recommended, or a value of corneal curvatures in a pupillary area should be recommended. In the present embodiment of the present invention, a result of the determinations that are combined is displayed on the monitor 50.

To be more specific, when astigmatism is equal to or more than 0.50 D in the corneal refractive powers K1 and K2 that correspond to a corneal curvature in a predetermined region of the examinee's eye, K2 is recommended. In addition, when astigmatism is less than 0.50 D and the deviation amount between the corneal center and the pupillary center is small, an average value of K1 and K2 is used.

In addition, when the deviation amount is large, an average power in the pupil that defines an average power obtained in a pupillary area of the corneal curvature distribution that is obtained as described above is recommended. In this case, the CPU 30 first extracts the pupillary area (the boundary portion between the iris and the pupil) by subjecting the anterior segment image that is obtained described above to image processing, and at the same time, extracts a corneal vertex position using a corneal bright point that is formed in the image, and then finds positional coordinates of a pupil rim relative to the corneal vertex position. Next, the CPU 30 makes the corneal vertex position that is extracted from a bright point of the corneal vertex that is formed in the placido ring image described above coincide with the corneal vertex position extracted from the anterior-segment image, and by superposing the pupil rims, calculates the positional coordinates of the pupil rim in the placido ring image. Then, the CPU 30 obtains corneal curvature distribution (corneal refractive power distribution) in the pupil based on information on the calculated position of the rim of the pupil position, and calculates an average value of the corneal curvature distribution.

Further, when a cursor of the mouse 41 is put on the average power in the pupil, the average value of K1 (a direction of the steepest meridian) and K2 (a direction of the flattest meridian), and the value of K2, the CPU 30 displays a comment that will be of reference to the surgeon in determining the corneal curvature. For example, when the value of K2 is designated, a comment "Correcting with a toric intraocular lens including an astigmatic component is recommended" is displayed.

After the input of all the items in the screening screen is completed as described above, and the mode shift button 74 is clicked on by the examiner operating the mouse 41, the CPU 30 shifts the screen to an ocular axial length measurement screen 110 as shown in FIG. 5.

The ocular axial length measurement screen 110 includes an area 111 arranged to display information on the apparatus that is used for the ocular axial length measurement, a measurement value capture button 112, and an area 113 arranged to display the obtained measurement result.

The used measurement apparatus, and the measurer (in a case where an ultrasonic ocular axial length measurement apparatus is selected) are displayed in the area 111. At this stage, when a pull-down button that is provided in a region where the measurement apparatus is displayed is clicked on with the mouse 41, a ultrasonic contact type ocular axial length measurement apparatus, or an optical coherence non-contact type ocular axial length measurement apparatus is displayed from a pull-down menu. Thus, clicking on an apparatus that concerns allows the used measurement apparatus to be selected. In this case, it is also preferable that apparatus selection should be made for each manufacture.

When a pull-down button that is provided in a region where the measurer name is displayed is clicked on if the ultrasonic ocular axial length measurement apparatus is selected in selecting the measurement apparatus, a list of previously-registered measures is displayed. Thus, clicking on a measurer that concerns allows the measurer to be selected. If a non-contact type measurement apparatus is selected, the pull-down button cannot be used.

In addition, when the ocular axial length measurement apparatus 20 is connected to the ophthalmic simulation apparatus 1 via LAN, allowing data transfer between the ocular axial length measurement apparatus 20 and the ophthalmic simulation apparatus 1, and the measurement value capture button 112 is clicked on with the mouse 41, the CPU 30 automatically obtains measurement values to store the measurement values in the memory unit 35, and at the same time, displays a result of the measurement in the area 113.

In addition, the measurement values of the ocular axial length, the depth of the anterior chamber, and a thickness of a crystalline lens that are expressed in the unit mm are displayed in this order in the area 113. In the area 113, when spinning buttons provided to areas arranged to display the measurement values are clicked on with the mouse 41, the measurement values are varied in predetermined steps. In this case, the measurement values can be input directly also with the keyboard 41. If an intraocular lens formula, in which the ocular axial length is used while the depth of the anterior chamber and the thickness of the crystalline lens are not used, (all the formulae except Camellin-Calossi formula in the present embodiment of the present invention) is selected in the screening mode described above, the CPU 30 allows the item of the ocular axial length to be input.

In addition, a correction result of the ocular axial length expressed in the unit mm is displayed in the area 113. In the area 113, when a spinning button provided to an area arranged to display the correction result is clicked on with the mouse 41, the measurement values are varied in predetermined steps. At this stage, if the ultrasonic ocular axial length measurement apparatus is selected, the CPU 30 carries out correction processing by adding a specific correction value that is set for every measurer to the input measurement value of the ocular axial length. This correction processing is carried out in order to correct a difference in measurement results that is made by difference among the measurers (e.g., difference in touching a probe among the measurers). If the correction value of the selected measurer is zero, the correction is regarded as no correction. In addition, if the non-contact type ocular axial length measurement apparatus is selected, a correction result that is subjected to correction processing is input so as to be compatible with a measurement vale obtained by a contact type ocular axial length measurement apparatus. Ii is also possible to set the non-contact type ocular axial length measurement apparatus as a standard.

The CPU 30 makes a determination as to whether the measurement values of the ocular axial length, the depth of the anterior chamber, and the thickness of the crystalline lens are normal. The determination is set as follows, for example. The ocular axial length is determined to be "normal when the ocular axial length is in the range from equal to or more than 22 mm to less than 28 mm, abnormality suspected when the ocular axial length is in the range from equal to or more than 20 mm to less than 22 mm/the range from equal to or more than 28 mm to less than 30 mm, and abnormal when the ocular axial length is in the range less than 20 mm/the range equal to or more than 30 mm". The depth of the anterior chamber is determined to be "normal when the depth of the anterior chamber is in the range from equal to or more than 3.5 mm to less than 4.5 mm, abnormality suspected when the depth of the anterior chamber is in the range from equal to or more than 2.0 mm to less than 3.5 mm/the range from equal to or more than 4.5 mm to less than 5.0 mm, and abnormal when the depth of the anterior chamber is in the range less than 2.0 mm/the range equal to or more than 30 mm". The thickness of the crystalline lens is determined to be "normal when the thickness of the crystalline lens is in the range from equal to or more than 2.5 mm to less than 4.0 mm, abnormality suspected when the thickness of the crystalline lens is in the range from equal to or more than 2.0 mm to less than 2.5 mm/the range from equal to or more than 4.0 mm to less than 5.0 mm, and abnormal when the thickness of the crystalline lens is in the range less than 2.0 mm/range equal to or more than 5.0 mm.

At this stage, when the measurement values (the ocular axial length, the depth of the anterior chamber, and the thickness of the crystalline lens) are determined to be normal, the areas arranged to display the measurement values are filled with a green color. When the measurement values are determined to be abnormality suspected, the areas arranged to display the measurement values are filled with a yellow color. When the measurement values are determined to be abnormal, the areas arranged to display the measurement values are filled with a red color.

After the ocular axial length measurement is completed as described above, and the mode shift button 74 is clicked on by the examiner operating the mouse 41, the CPU 30 shifts the screen to a data input screen 120 as shown in FIG. 6.

An area 121 arranged to display information to be used in the prescription of the intraocular lens is displayed in the data input screen 120. The area 121 displays intraocular pressure, a corneal center thickness, the number of corneal endothelial cells, naked eye vision, best-corrected visual acuity, information as to whether the subject eye is a dominant eye, information as to whether the examinee has experienced treatment for an enlarged prostate (a name of a treatment drug, and a duration of administration), information on the other eye (an SCA value, a VD value, and information as to whether cataract surgery has been performed on already), and other information. In this case, inputting and checking the measurement value for each displayed item can be performed on the screen. The CPU 30 makes a determination as to whether the measurement values of the intraocular pressure, the corneal center thickness, and the number of corneal endothelial cells are normal, and expresses a result of the determination by filing the areas arranged to display the measurement values with a green color (normal), a yellow color (abnormality suspected), or a red color (abnormal).

After the data-input operation is completed as described above and the mode shift button 74 is clicked on by the examiner operating the mouse 41, the CPU 30 shifts the screen to an IOL selection screen 130 as shown in FIG. 7.

The IOL selection screen 130 consists of an area 131 to an area 139. The area 131 is arranged to display information on a recommended intraocular lens among the intraocular lens models provided by the manufactures that have been previously registered in the memory unit 35. The area 132 is arranged to display information on determination of the power of the intraocular lens. The area 134 is arranged to display information on an IOL formula. The area 136 is arranged to display information on results of the measurement of the examinee's eye. The area 137 is arranged to display a K value that is used in calculating the power of the intraocular lens so to be selectable among a plurality of K values. The area 138 is arranged to display a map showing the corneal curvature distribution. The area 139 is arranged to display the anterior segment image.

A target post-operative residual refractive power (Target refractive power), target post-operative residual spherical aberration (Target residual S.A), and an ocular axial length value (AL) are displayed in the area 132. Further, IOL information on the selected intraocular lens (e.g., a manufacture name, a model name, a sphere power, an astigmatic axis power, and an astigmatic axial angle), and estimated post-operative residual spherical aberration (Post S.A) for each intraocular lens model are displayed in the area 131. In this case, a plurality of candidate intraocular lens models (a first candidate and a second candidate in the present embodiment of the present invention) selected by the CPU 30 are displayed. It is to be noted that the target post-operative residual spherical aberration TS defines a parameter to indicate post-operative spherical aberration of the examinee's eye that is desired by the examiner. The target post-operative residual refractive power and the target post-operative residual spherical aberration can be arbitrarily set with the use of the input unit 40. As for a method for the setting described above, and a method for calculating the estimated post-operative residual spherical aberration, refer to Japanese Unexamined Patent Application Publication No. 2009-34441.

Hereinafter, a description of a list DL will be provided. The CPU 30 specifies an intraocular lens that has a power that is close to a value calculated based on an IOL power that is calculated by substituting a selected post-operative target refractive power value (D), the measurement data of the examinee's eye (e.g., the corneal refractive power, and the ocular axial length), and the IOL information (e.g., the A constant, the estimated post-operative ACD, and the SF) into the predetermined IOL formula among the prepared intraocular lenses (in the present embodiment of the present invention, the intraocular lenses are prepared in 0.50 steps and the power information for each lens is registered in the memory unit 35).

Next, in order to obtain a post-operative refractive power value in the case of performing surgery to implant the specified ocular lens having the power as an estimated value Rx, the CPU 30 calculates the estimated value Rx (X = Rx) by assuming a portion of the target refractive power (D) in the predetermined IOL formula to be X, and substituting the value of the power of the specified intraocular lens, the measurement data on the examinee's eye, and the IOL information on the intraocular lens model into the predetermined IOL formula. That is, a formula for calculating an IOL power, and a formula for calculating the estimated residual refractive power (the estimated value Rx) are usually prepared as the IOL formula. In the case of using the toric intraocular lens, a spherical equivalent power of the selected toric intraocular lens is input, and an estimated residual spherical equivalent refractive power (an estimated residual spherical equivalent refractive diopter) is calculated. FIG.9 is an IOL formula in SRT formula that defines one example of the IOL formulae. The upper formula is for calculating an IOL power, and the lower formula is for calculating an estimated residual refractive power.

By using the IOL formula, the intraocular lens power with which prescription close to the target refractive power can be made is specified for each intraocular lens model selected as described above among the lens powers that are previously prepared, and at the same time, the post-operative eye refractive power value obtained by the specified intraocular lens power is calculated as the estimated value Rx. Then, the CPU 30 makes the obtained intraocular lens power correspond to the estimated value, and outputs the obtained intraocular lens power and the estimated value (see the shaded portion in the list DL).

In addition, the CPU 30 specifies the intraocular lens power to be prescribed for the examinee's eye with which prescription close to the refractive power can be made for each refractive power in a predetermined step (e.g., 0.50 D) within a predetermined range (e.g., ±2.00) relative to the set target refractive value among the intraocular lens powers that are previously prepared, and calculates the post-operative eye refractive power values obtained by the specified intraocular lens powers as the estimated values Rx. Then, the CPU 30 makes the obtained intraocular lens powers correspond to the estimated values, and outputs the obtained intraocular lens powers and the estimated values (see the unshaded portions in the list DL). In the list DL shown in FIG. 7, a result of the specification of the intraocular lens power and the calculation of the estimated value are shown so that the deviation amount with respect to the target refractive power corresponds to +2.00, +1.50, +1.00, +0.50, 0, -0.50, -1.00, -1.50, and -2.00.

After the intraocular lens selection is completed as described above and a mode shift button 150 is clicked on by the examiner operating the mouse 41, the CPU 30 shifts the screen to a seeing simulation screen 140 as shown in FIG. 8.

An area 141 arranged to display a result of a seeing simulation relating to the selected intraocular lens model is provided in the seeing simulation screen 140.

In this case, the CPU 30 carries out image processing to build as a simulation image the way of seeing a predetermined target in a case where an intraocular lens model is implanted into the examinee's eye based on the refractive power of the intraocular lens model and the corneal wavefront aberration of the cornea of the examinee's eye that are stored in the memory unit 35, and displays the simulation image where the intraocular lens model specified as described above is used, together with the name of the intraocular lens model on the monitor 50.

To be more specific, the CPU 30 calculates the wavefront aberration of the examinee's eye at the time when the arithmetic processing to add the prescribed values (the sphere power, the astigmatic power, and the astigmatic axial angle) and the spherical aberration of the selected intraocular lens to the wavefront aberration of the cornea of the examinee's eye that is obtained as described above is carried out, and obtains a point spread function (PSF) by using the obtained wavefront aberration. It is to be noted that in the case of using a toric intraocular lens, a simulation where the toric intraocular lens is implanted into the examinee's eye so as to cancel a corneal astigmatic component of the examinee's eye is carried out.

Next, the CPU 30 obtains a simulation image of how the predetermined target is formed on a retinal surface of the examinee's eye when the selected intraocular lens is implanted into the examinee's eye by subjecting the obtained PSF and the predetermined target (each item of entire ETDRS, entire ETDRS, entire ETDRS, and scenery) to image processing (convolution integration). At this stage, the CPU 30 obtains simulation images relating to the selected two intraocular lens models (i.e., intraocular lens models that are picked up as the candidates) to display the obtained images via the image processing unit 31. That is, the CPU 30 displays the simulation images eye side by side on the monitor 50 at the time when the plurality of selected intraocular lens models are implanted into the examinee's eye.

It is to be noted that the model names of the intraocular lens models displayed on the monitor 50 can be changed to the model names of the other intraocular lens models stored in the memory unit 35. Thus, when a model name of an intraocular lens model is changed, the CPU 35 displays the changed model name of the intraocular lens model on the monitor 50, and at the same time, changes a corresponding simulation image through image processing to display the changed simulation image on the monitor 50.

To be more specific, an IOL model name, a refractive power of an IOL (IOL pwr/Cylinder), an estimated residual refractive power Rx, a depth pACD of an estimated post-operative anterior chamber, an estimated post-operative refractive power (Sph/ Cyl/Axis/SE) of the entire eye, a Strehl ratio, an estimated post-operative residual spherical aberration (Residual S.A), and a simulation image are displayed for each intraocular lens in simulation displays 500 and 501. The simulation image consists preferably of a simulation image of the entire ETDRS, a simulation image of the detailed ETDRS, and a simulation image of the scenery.

When the area of the IOL model name is pulled down, the model names of the intraocular lenses that are arranged from the top in the order in which the estimated spherical aberrations of the intraocular lenses are closer to the target spherical aberration are displayed in list form. When a desired intraocular lens model is selected from the list, the seeing simulation corresponding to the selected model is displayed. In this manner, at least two intraocular lens models can be selected, and the simulation results of the intraocular lens models are displayed, which allows selection of the intraocular lens from the viewpoint of the examinee. It is also preferable that a spatial frequency characteristic MTF in a case where the selected intraocular lens model is implanted into the examinee's eye should be displayed in the seeing simulation described above (e.g., graph display with spatial frequency on the the horizontal axis and contrast sensitivity on the vertical axis) . It is to be noted that the MTF can be obtained by performing a Fourier transform on the point spread function PSF that is obtained from the wavefront aberration of the examinee's eye. In this manner, a more precise seeing simulation can be achieved.

After the intraocular lens selection is completed as described above and a print button (not illustrated) is clicked on by the examiner operating the mouse 41, the CPU 30 shifts the screen to a print screen (not illustrated) to print out the information on the selected intraocular lens from the printer 43.

<IOL post-operative simulation using an estimated residual refractive power obtained by an intraocular lens power formula>

FIG. 10 is a flow chart for illustrating a method for a seeing simulation according to the present embodiment of the present invention. The present method is for an IOL post-operative simulation using an estimated residual refractive power obtained by an intraocular lens power formula.

<Determination of irregular astigmatism>

The CPU 30 obtains an amount of irregular astigmatism based on the information such as the RMS of the cornea and the Zernike coefficient. The CPU 30 makes a determination as to whether or not the effect of correction by the toric intraocular lens is sufficient based on whether or not the obtained irregular astigmatism amount goes beyond an allowable range. The allowable range used for the determination is obtained preferably by experiment . When the irregular astigmatism amount goes beyond the allowable range, the CPU makes the announcement that the effect of correction by the toric intraocular lens is not sufficient (e.g., message display, and iconic display).

<Calculation of prescribed values of an intraocular lens>

When the power of the intraocular lens is determined, the following arithmetic processing is carried out. As described above, the CPU 30 calculates the sphere power of the intraocular lens using the designated intraocular lens power formula. The CPU 30 obtains an astigmatic power of the intraocular lens that corresponds to an astigmatic power (C) of the cornea of the examinee's eye. The CPU 30 obtains an axial angle of the intraocular lens at the time when the toric intraocular lens is implanted into the examinee's eye so as to cancel the corneal astigmatic component. In general, the toric intraocular lens is disposed such that the direction of the flattest meridian of the intraocular lens coincides with the direction of the steepest meridian of the cornea. Further, the CPU 30 obtains a spherical equivalent power of the intraocular lens based on the calculated sphere power and the astigmatic power of the intraocular lens. The above-described sphere power, astigmatic power, and spherical equivalent power define ideal powers to obtain a target refractive power, and the intraocular lenses that can be implanted to the examinee's eye are prepared in the predetermined step. When the intraocular lens that is customized so as to have an ideal power in order to correct the examinee's eye is manufactured, the intraocular lens can deal with the ideal power.

Based on the spherical equivalent power and the astigmatic power of the intraocular lens, the CPU 30 specifies a spherical equivalent power and an astigmatic power of a toric intraocular lens that can be implanted into the examinee's eye, the spherical equivalent power and the astigmatic power being close to the two power values of the intraocular lens, among the intraocular lens powers that are previously prepared in the predetermined step. In this manner, prescribed values (a sphere power, an astigmatic power, and an astigmatic axial angle) of the toric intraocular lens for the examinee's eye are determined.

Usually, at least two kinds of parameters of the spherical equivalent powers and the astigmatic powers of the toric intraocular lenses of the manufactures' models are already known. In addition, the spherical equivalent powers and the astigmatic powers of the toric intraocular lenses of the manufactures' models are prepared in the predetermined step (e.g., 0.75 D step) . The information on the toric intraocular lenses that actually exist is stored for each manufacture's model in the memory unit 35. It is preferable that based on a complete spherical equivalent power, a complete astigmatic power, and spherical aberration of the intraocular lens, the CPU 30 should select the power of the intraocular lens that is closest to the three parameters among the intraocular lens powers previously prepared. The CPU 30 specifies the power of the intraocular lens based on an operation signal sent from the input unit 40 operated by the examiner.

The CPU 30 obtains an estimated residual sphere power (SE) at the time when the specified intraocular lens is prescribed for the examinee's eye with the use of the IOL formula arranged to calculate the estimated residual refractive power. The CPU 30 outputs the estimated residual sphere power (SE) on the monitor 50.

<Calculation of a post-operative corneal refractive power>

It is also preferable that considering surgically induced astigmatism to be caused, the CPU 30 should obtain a post-operative corneal refractive power by combining a pre-operative corneal astigmatic component (SCA) and induced astigmatism component. The induced astigmatism is caused by corneal incision during surgery, for example . The induced astigmatism component is previously obtained preferably by experiment, and stored in the memory unit 35. The generation state of the induced astigmatism component differs with the incision position on the cornea, so that the induced astigmatism component is obtained in accordance with information on the incision position that is input via the input unit 40.

< Lens expression method by a refractive power matrix>

A refractive power and principal meridian directions of a lens of which the refractive power differs with the principal meridian directions such as a toric lens are expressed by rows and columns of a two-by-two matrix (a refractive power matrix). By using the refractive power matrix, the combined refractive power and the principal meridian directions at the time when the plurality of toric lenses are disposed in an arbitrary principal meridian direction and at an arbitrary axial position (a distance between the lenses) can be obtained.

A refractive power matrix D is expressed by a determinant of a two-by-two matrix.

[Expression 1]

$$\boldsymbol{D} = \begin{bmatrix} D_{xx} & D_{xy} \\ D_{yx} & D_{yy} \end{bmatrix} \qquad \text{Formula 1}$$

The components of the rows and columns relating to the toric lens of SCA display is defined by the following formula.

[Expression 2]

$$D_{xx} = S + C\ \sin^2\theta \qquad \text{Formula 2}$$

$$D_{xy} = D_{yx} = -C\ \sin\theta\cos\theta \qquad \text{Formula 3}$$

$$D_{yy} = S + C\ \cos^2\theta \qquad \text{Formula 4}$$

$$\boldsymbol{D} = \begin{bmatrix} S + C\ \sin^2\theta & -C\ \sin\theta\cos\theta \\ -C\ \sin\theta\cos\theta & S + C\ \cos^2\theta \end{bmatrix} \qquad \text{Formula 5}$$

The above formula becomes same as the following formula when the powers of the two principal meridian directions that are perpendicular to each other are assumed to be DS (S), and DT (S+C), and the angle of the DS (the angle of the S) is assumed to be θ. It is to be noted that c = dt - ds, s = ds (ds > dt) .

[Expression 3]

$$D_{xx} = D_T\ \sin^2\theta + D_S\cos^2\theta \qquad \text{Formula 6}$$

$$D_{xy} = D_{yx} = (D_S - D_T)\sin\theta\cos\theta \qquad \text{Formula 7}$$

$$D_{yy} = D_S\ \sin^2\theta + D_T\cos^2\theta \qquad \text{Formula 8}$$

$$D = \begin{bmatrix} D_T \sin^2\theta + D_S \cos^2\theta & (D_S - D_T)\sin\theta\cos\theta \\ (D_S - D_T)\sin\theta\cos\theta & D_S \sin^2\theta + D_T \cos^2\theta \end{bmatrix}$$ Formula 9

Transformation from the refractive power matrix D to the SCA display is expressed by the following formula (C is minus display).
[Expression 4]

$$C = -\sqrt{(D_{xx} + D_{yy})^2 - 4 \times detD} \quad (detD = D_{xx}D_{yy} - D_{xy}D_{yx})$$ Formula 10

$$S = \frac{1}{2}(D_{xx} + D_{yy} - C)$$ Formula 11

$$tan\theta = \frac{S - D_{xx}}{D_{xy}} \quad or \quad tan\theta = \frac{D_{yx}}{S - D_{yy}}$$ Formula 12

$$\begin{cases} If\ D_{xy} = 0\ and\ S - D_{yy} \neq 0\ then\ \theta = 0^\circ \\ If\ D_{xy} = 0\ and\ S - D_{yy} = 0\ then\ \theta = 90^\circ \end{cases}$$

* For example, limitation is made as follows in order to round fractions in numerical calculation. The range of the limitation is arbitrary, and is determined from numerical precision by the program. Formula 13

$$\text{If} \left|D_{xy}\right| < 10^{-12}\ then\ D_{xy} = 0$$

$$\text{If} \left|S - D_{yy}\right| < 1E - 12\ then\ S - D_{yy} = 0$$

A rotation matrix R, and an inverse matrix $R^{-1}$ of the rotation matrix R becomes the following determinant when a rotation angle is assumed to be ϕ.
[Expression 5]

$$R = \begin{bmatrix} \cos\varphi & -\sin\varphi \\ \sin\varphi & \cos\varphi \end{bmatrix} \qquad R^{-1} = \begin{bmatrix} \cos\varphi & \sin\varphi \\ -\sin\varphi & \cos\varphi \end{bmatrix}$$ Formula 14

When the toric lens that is displayed in a given refractive power matrix D is rotated by the ϕ around the optical axis center, a refractive power matrix D' after the rotation is obtained by the following formula using the R and the $R^{-1}$.
[Expression 6]

$$D' = RDR^{-1}$$

$$= \begin{bmatrix} \cos\varphi & -\sin\varphi \\ \sin\varphi & \cos\varphi \end{bmatrix} \begin{bmatrix} D_{xx} & D_{xy} \\ D_{yx} & D_{yy} \end{bmatrix} \begin{bmatrix} \cos\varphi & \sin\varphi \\ -\sin\varphi & \cos\varphi \end{bmatrix}$$

$$= \begin{bmatrix} D_{xx} \sin^2\varphi + D_{yy} \cos^2\varphi & (D_{xx} - D_{yy})\sin\varphi\cos\varphi \\ (D_{xx} - D_{yy})\sin\varphi\cos\varphi & D_{yy} \sin^2\varphi + D_{xx} \cos^2\varphi \end{bmatrix}$$ Formula 15

<Combination (close contact) of the toric lenses>

Hereinafter, the refractive power matrix D is expressed as [D] for the sake of simplicity. The combined refractive power matrix [D] at the time when two thin toric lenses having a refractive power matrix [D1] and a refractive power matrix [D2] are brought into close contact with and superposed on each other, which are like the cornea and the post-operative induced astigmatism, can be expressed by the following formula.
[Expression 7]

$$[D] = [D_1] + [D_2] \qquad \text{Formula 16}$$

In the same manner, when the number of the lenses is n, the combined refractive power matrix [D] can be expressed by the following formula.
[Expression 8]

$$[D] = [D_1] + [D_2] + [D_3] + \cdots + [D_n] = \sum_{1}^{n} [D_n] \qquad \text{Formula 17}$$

<Calculation of a refractive power of the entire eye>

The CPU 30 calculates a refractive power (a refractive diopter: a sphere power (sph), an astigmatic power (cyl), and an astigmatic axial angle (axis)) of the entire eye by using the post-operative corneal refractive power (the corneal refractive diopter: a sphere power, an astigmatic power, and an axial angle) of the examinee's eye, the refractive power (the refractive diopter of the intraocular lens: a sphere power, an astigmatic power, and an axial angle) of the toric intraocular lens to be prescribed for the examinee's eye, a distance between the cornea and the intraocular lens (a depth ACD of an estimated post-operative anterior chamber), and a value L of the ocular axial length of the examinee's eye that is obtained by the ocular axial length measurement apparatus 20.

For example, the CPU 30 combines the corneal refractive power and the refractive power of the intraocular lens that is apart from the cornea into the eye by the ACD, and calculates the refractive power of the entire eye assuming that the retina that is located at a position of the ocular axial length is an image forming position. For example, 5 mm is used as a default of the ACD.

<Combination of the toric lenses>

When the refractive power at the time when the thin toric lenses are apart from each other as the post-operative cornea and the intraocular lens are is taken into consideration, there are a principal point refractive power that is the combined refractive power of the thin toric lenses, and a vertex refractive power that defines a refractive power at the time of emission of the image-side lens. Inverse matrixes of the refractive power of the principal point and the vertex refractive power respectively define a principal point focal point distance and a vertex focal length. In the normal geometric optics, the principal point focal point distance and the vertex focal point distance respectively correspond to a combined focal length, and a back focal length (BFL).

A vertex refractive power [U2'] and a vertex focal length [Bf2'] at the time when the thin toric lenses are apart from each other are obtained.

[Un]: a vergence matrix of an incident light bundle
(vergence matrix)
[Un'] : a vergence matrix of an exit light bundle (vergence matrix')
[Dn]: a refractive power matrix of a lens (power matrix) Dn: an air-converted distance (for example, let physical lengths of a lens 1 and a lens 2 represent t1, and let a refraction index of a medium between the lenses represent n1, d1 = t1/n1)

The following relation holds in FIG. 11.
[Expression 9]

$$[U_1] = \frac{1}{d_{0(-)}}[E] = \frac{1}{d_{0(-)}}\begin{bmatrix}1 & 0\\0 & 1\end{bmatrix}$$ Formula 18

If parallel light enters,
[Expression 10]

$$[U_1] = \begin{bmatrix}0 & 0\\0 & 0\end{bmatrix}$$ Formula 19

An imaging formula of the lens 1
[Expression 11]

$$[U_1'] = [U_1] + [D_1]$$ Formula 20

Conversion from the [U1'] to the [U2]
[Expression 12]

$$[U_2] = [U_1']([E] - d_1[U_1'])^{-1}$$ -1 means an inverse matrix Formula 21

An imaging formula of the lens 2
[Expression 13]

$$[U_2'] = [U_2] + [D_2]$$ Formula 22

When the formula is rearranged by isolating the [U2'],
[Expression 14]

$$[U_2'] = ([U_1] + [D_1])\{[E] - d_1([U_1] + [D_1])\}^{-1} + [D_2]$$ Formula 23

is held, and [U2'] becomes an image-side vertex refractive power matrix.

In addition, an image-side vertex focal length matrix [Bf'] can be obtained by obtaining an inverse matrix of the [U2'] as
[Expression 15]

$$[\mathrm{Bf}'] = [U_2']^{-1}$$ Formula 24

It is to be noted that when the components of the matrix [Bf'] are respectively substituted into the formula 9, the formula 10, and the formula 11 as they are, a difference ΔBf' of a vertex focal length between the direction of the steepest meridian and the direction of the flattest meridian, a vertex focal length Bf' (S+C) in the direction of the flattest meridian, and an axis in the direction of the flattest meridian (in other words, an angle up to S+C) are respectively obtained. In addition, a vertex focal length in the direction of the steepest meridian is defined as follows:
[Expression 16]

$$\mathrm{Bf}_{(S)} = \mathrm{Bf}_{(S+C)} + \Delta\mathrm{Bf}_{(-)}$$ Formula 25

These values define values in a medium having a refractive power of 1, and are expressed in the unit meter. If these values define values in a medium having a refraction index of n, the ΔBf, the Bf(S+C), and the Bf(S) may only be multiplied by n. It is to be noted that a refraction index in the air is set to be 1, and a refraction index in aqueous humor is set to be 1.336.

In case it helps, this explanation is summarized in the following formulae.

[Expression 17]

$$\Delta Bf' = -\sqrt{(D_{xx} + D_{yy})^2 - 4 \times detD} \quad (detD = D_{xx}D_{yy} - D_{xy}D_{yx}) \qquad \text{Formula 26}$$

$$Bf'_{(S+C)} = \frac{1}{2}(D_{xx} + D_{yy} - \Delta Bf') \qquad \text{Formula 27}$$

$$Bf'_{(S)} = Bf'_{(S+C)} + \Delta Bf'_{(-)}$$

$$tan\theta = \frac{S - D_{xx}}{D_{xy}} \quad or \quad tan\theta = \frac{D_{yx}}{S - D_{yy}} \qquad \text{Formula 28}$$

$$\begin{cases} If\ D_{xy} = 0\ and\ S - D_{yy} \neq 0\ then\ \theta = 0^\circ \\ If\ D_{xy} = 0\ and\ S - D_{yy} = 0\ then\ \theta = 90^\circ \end{cases}$$

**[0117]** * For example, limitation is made as follows in order to round fractions in numerical calculation. The range of the limitation is arbitrary, and is determined from numerical precision by the program.

$$If\ |D_{xy}| < 10^{-12}\ then\ D_{xy} = 0$$

$$If\ |S - D_{yy}| < 10^{-12}\ then\ S - D_{yy} = 0$$

Also in the case of using two or more toric lenses, by repeating the above [imaging formula, conversion formula], a vertex refractive power matrix can be obtained. In addition, a vertex focal length matrix can be obtained by obtaining an inverse matrix of the vertex refractive power matrix.

The CPU 30 calculates a refractive power of the entire eye assuming that a retina that is located at a position of the ocular axial length of the examinee's eye is an image forming position. If the sph is calculated based on the difference Δ in the optical axis direction of the retina, a vertex focal length position in the direction of the flattest meridian is considered as a standard. Let an ocular axial length of an eye ball represent L, and a depth of an estimated post-operative anterior chamber represent ACD, the difference Δ (a physical length) in the optical axis direction of the retina is expressed as follows.

[Expression 18]

$$\Delta = L - (ACD + B_F) \qquad \text{Formula 29}$$

A formula arranged to convert the Δ into the sph is developed. A formula arranged to compute the sph is expressed as follows.

[Expression 19]

$$sph = \frac{1}{\left(\frac{B_F + \Delta}{1000 \cdot n_2}\right)} - \frac{1}{\left(\frac{B_F}{1000 \cdot n_2}\right)} = \frac{-1000 \cdot n_2 \cdot \Delta}{B_F(B_F + \Delta)} \qquad \text{Formula 30}$$

Thus, when this formula is rearranged by isolating Δ, the following formula helds.
[Expression 20]

$$\Delta = \frac{-sph \cdot B_F^{2}}{sph \cdot B_F + 1000 \cdot n_2} \quad \text{Formula 31}$$

$$\text{If } \Delta > 0 \text{ then } sph < 0$$
$$\text{If } \Delta < 0 \text{ then } sph > 0 \quad \text{Formula 32}$$

Thus, the sph is obtained as follows.
[Expression 21]

$$sph = \frac{-1000 \cdot n_2 \cdot \Delta}{B_F(B_F + \Delta)} \quad \text{Formula 33}$$

The astigmatic power cyl, and the astigmatic axial angle axis are obtained by the formula 10 and the formula 11.

<Comparison between the estimated residual refractive power and the refractive power of the entire eye>

The CPU 30 calculates the spherical equivalent power of the entire eye that corresponds to the sphere power and the astigmatic axis power in the refractive power of the entire eye.

The CPU 30 compares the estimated residual sphere power SE with the spherical equivalent power of the entire eye. The CPU 30 varies the ACD value contained in the formula (see the Formula 28) arranged to calculate the refractive power of the entire eye in a step-by-step manner, and finds a pACD value such that the estimated residual sphere power SE corresponds with the spherical equivalent power of the entire eye. For example, the CPU 30 varies at any time the ACD value in the formula from the default in the step of 0.1 mm, and calculates by feedback calculation a depth pACD of an estimated post-operative anterior chamber at the time when the estimated residual refractive power obtained based on the IOL formula is obtained. The CPU 30 outputs thus-obtained pACD value (the depth of the estimated post-operative anterior chamber) on the monitor 50. The CPU 30 obtains the refractive power (a refractive diopter: a sphere power (sph), an astigmatic power (cyl), and an astigmatic axial angle (axis)) of the entire eye that is calculated by using the pACD value as the post-operative refractive power of the entire eye that is calculated by the IOL formula by using the estimated residual refractive power.

The IOL power formula defines a formula with which a post-operative estimated residual sphere power SE can be calculated; however, a sphere power, an astigmatic power, and an astigmatic axial angle are required to carry out a post-operative simulation. To be specific, while a post-operative estimated residual sphere power SE can be calculated by the IOL power formula by substituting a spherical equivalent power of an intraocular lens into the IOL power formula, the contents (a sphere power, and an astigmatic power) of the spherical equivalent values are not calculated by the IOL power formula.

Thus, by carrying out arithmetic processing based on the premise that the estimated residual sphere power SE calculated by the IOL power formula indicates a post-operative spherical equivalent power, a residual refractive power (a refractive diopter: a sphere power (sph), an astigmatic power (cyl), and an astigmatic axial angle (axis)) of the entire eye that corresponds with the result by the IOL power formula can be obtained.

<Calculation of post-operative correction data based on the refractive power of the entire eye>

The CPU 30 substitutes the post-operative refractive power of the entire eye that is obtained as described above into the SCA in a quadratic expression of the Zernike's polynomial, and calculates a coefficient of the quadratic expression that correspond to the refractive power of the entire eye. A wavefront (W) is expressed in the following formula by the Zernike's polynomial.

[Expression 22]

$$W(\rho,\theta)=\sum_{n}\sum_{m} C_n^m Z_n^m(\rho,\theta)$$

$$Z_n^m(\rho,\theta)=\begin{cases} N_n^m R_n^{|m|}(\rho)\sin(|m|\theta)\,;(0<m) \\ N_n^m R_n^{|m|}(\rho)\cos(m\theta)\,;(0\geq m) \end{cases}$$

$$R_n^{|m|}(\rho)=\sum_{s=0}^{\frac{n-|m|}{2}} \frac{(-1)^s(n-s)!}{s!\,[0.5(n+|m|)-s]!\,[0.5(n-|m|)-s]!}\rho^{n-2s}$$

$$N_n^m=\sqrt{\frac{2(n+1)}{1+\delta_{m0}}}$$

$$\delta_{m0}=1 \text{ if } m=0,\ \delta_{m0}=0 \text{ if } m \text{ is not } 0$$

$C_n^m$ Zernike Coefficient, $N_n^m$ normalized coefficient ρ exit pupil radius, θ angle, R defines a radius (mm) of a pupil diameter to be analyzed

A formula arranged to obtain a low-order Zernike's coefficient from the post-operative estimated low-order aberration sph, cyl, and axis of the entire eye is expressed in the following formula. A high-order Zernike's coefficient is obtained by using the aberration measurement value of a corneal anterior surface that is obtained by the corneal shape measurement apparatus, and spherical aberration can be obtained by substituting again an estimated value into the formula.

[Expression 23]

$$C_2^{-2}=\sqrt{15}\,C_4^{-2}-2\sqrt{21}\,C_6^{-2}+\frac{\text{cyl}\,R^2\sin(2\,\text{axis})}{4\sqrt{6}\lambda},$$

$$C_2^{+2}=\sqrt{15}\,C_4^{+2}-2\sqrt{21}\,C_6^{+2}+\frac{\text{cyl}\,R^2\cos(2\,\text{axis})}{4\sqrt{6}\lambda}$$

axis : the flattest meridian

$$C_2^{-2}=\sqrt{15}\,C_4^{-2}-2\sqrt{21}\,C_6^{-2}-\frac{\text{cyl}\,R^2\sin(2\,\text{axis})}{4\sqrt{6}\lambda},$$

$$C_2^{+2}=\sqrt{15}\,C_4^{+2}-2\sqrt{21}\,C_6^{+2}-\frac{\text{cyl}\,R^2\cos(2\,\text{axis})}{4\sqrt{6}\lambda}$$

axis: the steepest meridian

$$C_2^0=\frac{1}{4\sqrt{3}}\left[\begin{matrix}(12\sqrt{5}C_4^0-24\sqrt{7}C_6^0)\pm \\ 2\left\{\left(\sqrt{6}C_2^{-2}-3\sqrt{10}C_4^{-2}+6\sqrt{14}C_6^{-2}\right)^2+\left(\sqrt{6}C_2^{+2}-3\sqrt{10}C_4^{+2}+6\sqrt{14}C_6^{+2}\right)^2\right\}^{\frac{1}{2}}\end{matrix}\right]-\frac{R^2\,\text{sph}}{4\sqrt{3}\lambda}$$

When represented in "-" cylinder, "±" is expressed as "+", When represented in "+" cylinder, "±" is expressed as "-".

$$C_0^0 = \sqrt{3}C_2^0 - \sqrt{5}C_4^0 + \sqrt{7}C_6^0$$

The CPU 30 calculates a coefficient of the Zernike's polynomial that represents wavefront aberration at the time when the sphere power (sph), the cylindrical power (cyl), and the angle (axis) of the entire eye that are estimated after the IOL is implanted into the examinee's eye, and the amount of the spherical aberration of the intraocular lens are added to the high-order component of the corneal wavefront aberration.

<Inverse operation of the wavefront aberration data>

The CPU carries out an inverse operation of the wavefront aberration data by using a quadratic Zernike's coefficient that corresponds to the post-operative refractive power of the entire eye, a third or more degree Zernike's coefficient that corresponds to the high-order aberration of the cornea of the examinee's eye, and a high-order Zernike's coefficient that corresponds to the spherical aberration amount of the intraocular lens. The refractive power of the entire eye corresponds to a low-order aberration component of the wavefront aberration. The high-order aberration of the cornea of the examinee's eye corresponds to an aberration component that is obtained by subtracting the component corresponding to the corneal refractive power from the entire aberration of the cornea of the examinee's eye.

The CPU 30 obtains a point spread function (PSF) by using the wavefront aberration data that is calculated from the inverse operation. The Zernike's polynomial is a representative example of the polynomial arranged to approximate the wavefront aberration data. It is needless to say that the polynomial arranged to approximate the wavefront aberration data is not limited to the Zernike's polynomial as long as the polynomial is arranged to approximate the wavefront aberration.

<Creation of a simulation image>

The CPU 30 obtains a point spread function (PSF) by using the wavefront aberration data that is calculated from the inverse operation. The CPU 30 subjects the obtained PSF and a predetermined target (e.g., an ETDRS target, a resolution chart, and a scenery chart) to image processing (convolution integration). The CPU 30 can obtain a simulation image of how the predetermined target is formed on a retinal surface of the examinee's eye when the selected intraocular lens is prescribed for the examinee's eye by the selected toric intraocular lens.

The simulation image obtained as described above is an image that is simulated in accordance with the estimated residual spherical equivalent power sphere power obtained by the intraocular lens power formula, and therefore is not inconsistent with an intraocular lens power formula that is developed based on theory or experience. Thus, even if detailed design values of the intraocular lenses of the variety of manufacturers are not known, a favorable simulation image can be obtained by using the known intraocular lens data (the spherical equivalent power, and the astigmatic power).

<Modification>

While the simulation image is obtained in the above description by finding the refractive power of the entire eye by varying the ACD so as to correspond with the estimated residual spherical equivalent power that is obtained by the intraocular lens power formula, the present invention is not limited to this manner. For example, the CPU 30 obtains the sphere power and the astigmatic power of the entire eye by obtaining the residual refractive power that corresponds to the direction of the steepest meridian of the intraocular lens and the residual refractive power that corresponds to the direction of the flattest meridian of the intraocular lens with the use of the intraocular lens formula arranged to obtain the residual refractive power. In addition, the CPU 30 calculates the astigmatic axial angle of the entire eye based on the position of the intraocular lens, the position where the astigmatic axial angle of the cornea and the corneal astigmatism can be canceled. Then, the CPU 30 obtains the simulation image by using the calculated refractive power (the sphere power, the astigmatic power, and the astigmatic axial angle) of the entire eye.

It is to be noted that while the case where the toric intraocular lens is prescribed is explained in the above description, the following manner can be applied to the simulation that uses the residual refractive power obtained by the intraocular lens power formula even if an intraocular lens that does not have the function of astigmatic correction is used. In this case, the CPU 30 obtains the simulation image by using the sphere power of the intraocular lens and the residual sphere power that is obtained by the intraocular lens power formula.

The foregoing description of the present embodiment of the present invention has been presented for purposes of illustration and description. It is not intended to be exhaustive or to limit the invention to the precise form disclosed. The embodiments chosen and described in order to explain the principles of the invention and its practical application to enable one skilled in the art to utilize the invention in various embodiments and with various modifications as are suited to the particular use contemplated. It is intended that the scope of the invention be defined by the claims appended hereto.

**Claims**

1. An ophthalmic measurement program that is carried out in an ophthalmic simulation apparatus arranged to output a simulation image (141) of a retinal image of an ex-aminee's eye at a time when an intraocular lens is implanted in the eye, and arranged to make the ophthalmic simulation apparatus carry out, by using a processor included in the ophthalmic simulation apparatus, an aberration data obtaining step of obtaining data on a wavefront aberration of the cornea of the examinee's eye that is measured with the use of a corneal shape measurement apparatus (10) arranged to measure a corneal shape of the eye by projecting a placido ring target onto the cornea of the eye and photo-receiving reflection light of the placido ring target or by using a principle of optical interference, an image creating step of creating the simulation image (141) based on the wavefront aberration data, and a display step of displaying the created simulation image on a monitor, **characterized in that**:

   (a) the ophthalmic measurement program comprises a post-operative calculation step of calculating a post-operative sphere power, a post-operative astigmatic power, and a post-operative astigmatic axial angle of the entire eye based on an estimated post-operative refractive power of the examinee's eye that is calculated by a predetermined intraocular lens formula, and
   (b) the image creating step comprises (b1) calculating coefficients of a polynomial that comprises the post-operative sphere power, the post-operative astigmatic power, and the post-operative astigmatic axial angle that are calculated in the post-operative calculation step, and a high-order aberration of a third or more degree of the corneal wavefront aberration that is obtained in the aberration data obtaining step, the polynomial being arranged to approximate the wavefront aberration data of the entire eye, (b2) performing an inverse operation on the wavefront aberration data of the entire eye with the use of the calculated coefficients of the polynomial, and (b3) creating the simulation image (141) based on the wavefront aberration data on which the inverse operation is performed.

2. The ophthalmic measurement program according to claim 1,
wherein the image creating step further comprises calculating a coefficient of a polynomial that comprises a spherical aberration amount of the intraocular lens, the polynomial being arranged to approximate the wavefront aberration data of the entire eye.

3. The ophthalmic measurement program according to claim 1 or 2,
wherein the post-operative calculation step comprises calculating the post-operative sphere power, the post-operative astigmatic power, and the post-operative astigmatic axial angle of the entire eye based on an estimated post-operative spherical equivalent refractive power that is calculated by the predetermined intraocular lens formula arranged to calculate the post-operative refractive power.

4. The ophthalmic measurement program according to any one of claims 1 to 3, wherein the intraocular lens comprises a toric intraocular lens, and
wherein the post-operative calculation step comprises calculating the post-operative sphere power, the post-operative astigmatic power, and the post-operative astigmatic axial angle of the entire eye assuming that the toric intraocular lens is disposed to cancel corneal astigmatism.

5. The ophthalmic measurement program according to any one of claims 1 to 4,
wherein the post-operative calculation step comprises calculating the post-operative sphere power, the post-operative astigmatic power, and the post-operative astigmatic axial angle of the entire eye under a condition that a post-operative refractive power of the entire eye that is calculated based on a combined refractive power of a corneal refractive power of the examinee's eye and a refractive power of the intraocular lens to be implanted into the examinee's eye, and an ocular axial length of the examinee's eye corresponds to the estimated post-operative refractive power of the examinee's eye that is calculated by the predetermined intraocular lens formula.

6. The ophthalmic measurement program according to any one of claims 1 to 4,
wherein the post-operative calculation step comprises
combining a corneal refractive power of the examinee's eye, and a refractive power of the intraocular lens that is apart from the cornea into the eye by a depth ACD of an estimated post-operative anterior chamber, and calculating the post-operative sphere power, the post-operative astigmatic power, and the post-operative astigmatic axial angle of the entire eye assuming that a retina that is located at a position of the ocular axial length of the examinee's eye is an image forming position.

**7.** The ophthalmic measurement program according to any one of claims 1 to 6,
wherein the post-operative calculation step comprises
varying a depth ACD of an estimated post-operative anterior chamber that a formula arranged to obtain the post-operative sphere power, the post-operative astigmatic power, and the post-operative astigmatic axial angle of the entire eye comprises, and
calculating the post-operative sphere power, the post-operative astigmatic power, and the post-operative astigmatic axial angle of the entire eye by using the depth ACD of the estimated post-operative anterior chamber at which the estimated post-operative refractive power that is calculated by the predetermined intraocular lens formula corresponds to the post-operative sphere power of the entire eye.

**8.** The ophthalmic measurement program according to any one of claims 1 to 6,
wherein the post-operative calculation step comprises
varying a depth ACD of an estimated post-operative anterior chamber that a formula arranged to obtain the post-operative sphere power, the post-operative astigmatic power, and the post-operative astigmatic axial angle of the entire eye comprises, and
calculating the post-operative sphere power, the post-operative astigmatic power, and the post-operative astigmatic axial angle of the entire eye by using the depth ACD of the estimated post-operative anterior chamber at which an estimated post-operative spherical equivalent refractive power that is calculated by the predetermined intraocular lens formula corresponds to a post-operative spherical equivalent power of the entire eye.

**9.** The ophthalmic measurement program according to claim 8,
wherein the display step comprises displaying on the monitor the depth ACD of the estimated post-operative anterior chamber at which the estimated post-operative spherical equivalent refractive power corresponds to the post-operative spherical equivalent power of the entire eye.

**10.** Theophthalmic measurement program according to any one of claims 1 to 9,
wherein the post-operative refractive power of the entire eye is calculated based on a combined refractive power of a corneal refractive power of the examinee's eye and a sphere power of the intraocular lens to be implanted into the examinee's eye, and an ocular axial length of the examinee's eye.

**11.** The ophthalmic measurement program according to any one of claims 1 to 10, further comprising a step of calculating the estimated post-operative refractive power of the examinee's eye by substituting parameters relating to a corneal shape of the examinee's eye, an ocular axial length of the examinee's eye, and an intraocular lens to be implanted into the examinee's eye in the predetermined intraocular lens formula.

**12.** The ophthalmic measurement program according to any one of claims 1 to 11,
wherein the predetermined intraocular lens formula comprises any one of SRK formula, SRK II formula, BINKHORST formula, HOFFER-Q formula, HOLLADAY formula, and HAIGIS formula.

**13.** An ophthalmic simulation apparatus that is arranged to output a simulation image (141) of a retinal image of an examinee's eye at a time when an intraocular lens is implanted in the eye, and comprises aberration data obtaining means adapted to obtain a wavefront aberration data of the cornea of the examinee's eye that is measured with the use of a corneal shape measurement apparatus (10) arranged to measure a corneal shape of the eye by projecting a placido ring target onto the cornea of the eye and photo-receiving reflection light of the placido ring target or by using a principle of optical interference, image creating means adapted to create the simulation image (141) based on the wave-front aberration data, and display control means adapted to display the created simulation image on a monitor, **characterized in that**:

(a) the ophthalmic simulation apparatus comprises a post-operative calculation means adapted to calculate a post-operative sphere power, a post-operative astigmatic power, and a post-operative astigmatic axial angle of the entire eye based on an estimated post-operative refractive power of the examinee's eye that is calculated by a predetermined intraocular lens formula, and
(b) the image creating means is arranged (b1) to calculate coefficients of a polynomial that comprises the post-operative sphere power, the post-operative astigmatic power, and the post-operative astigmatic axial angle that are calculated by the post-operative calculation means, and high-order aberration of a third or more degree of the corneal wavefront aberration that is obtained in the aberration data obtaining means, the polynomial being arranged to approximate the wavefront aberration data of the entire eye, (b2) perform an inverse operation on the wavefront aberration data of the entire eye with the use of the calculated coefficients of the polynomial, and (b3) create the simulation image (141) based on the wavefront aberration data on which the inverse operation is performed.

## Patentansprüche

**1.** Ophthalmologisches Messprogramm, das in einer ophthalmologischen Simulationseinrichtung ausgeführt wird, die vorgesehen ist, um ein Simulationsbild (141) eines Retinabilds eines Auges einer zu untersuchenden Person auszugeben, wenn eine Intraokularlinse in dem Auge implantiert ist, und die vorgesehen ist, um zu bewirken, dass die ophthalmologische Simulationseinrichtung, unter Verwendung eines Prozessor, der in der ophthalmologische Simulationseinrichtung enthalten ist, einen Aberrationsdaten-Gewinnungsschritt zum Gewinnen von Daten einer Wellenfrontaberration der Hornhaut des Auges der zu untersuchenden Person, die mit Hilfe einer Hornhautform-Messeinrichtung (10) gemessen wird, die vorgesehen ist, um eine Hornhautform des Auges durch Projizieren eines Placidoringziels auf die Hornhaut des Auges und Empfangen eines Reflexionslichts des Placidoringziels oder unter Verwendung optischer Interferenz zu messen, einen Bilderzeugungsschritt zum Erzeugen des Simulationsbilds (141) auf der Grundlage der Wellenfront-Aberrationsdaten und einen Anzeigeschritt zum Anzeigen des erzeugten Simulationsbilds auf einem Monitor durchzuführen, **dadurch gekennzeichnet, dass**:

(a) das ophthalmologische Messprogramm einen post-operativen Berechnungsschritt zum Berechnen einer port-operativen, sphärischen Brechkraft, einer post-operativen, astigmatischen Brechkraft und eines post-operativen, axialen Astigmatismuswinkels des gesamten Auges auf der Grundlage einer geschätzten, post-operativen Brechkraft des Auges der zu untersuchenden Person, die durch eine vorbestimmte Intraokularlinsenformel berechnet wird, umfasst, und

(b) der Bilderzeugungsschritt (b1) ein Berechnen von Koeffizienten eines Polynoms, das die post-operative sphärische Brechkraft, die post-operative astigmatische Brechkraft und den post-operativen axialen Astigmatismuswinkel, die in dem post-operativen Berechnungsschritt berechnet werden, umfasst, und einer Aberration dritter oder höherer Ordnung der Hornhautwellenfrontaberration, die in dem Aberrationsdaten-Gewinnungsschritt gewonnen wird, wobei das Polynom dazu geeignet ist, die Wellenfront-Aberrationsdaten des gesamten Auges zu approximieren, (b2) ein Ausführen einer inversen Operation an den Wellenfront-Aberrationsdaten des gesamten Auges unter Verwendung der berechneten Koeffizienten des Polynoms und (b3) ein Erzeugen des Simulationsbilds (141) auf der Grundlage der Wellenfront-Aberrationsdaten, auf die die inverse Operation ausgeführt wird, umfasst.

**2.** Ophthalmologisches Messprogramm nach Anspruch 1,
wobei der Bilderzeugungsschritt ferner ein Berechnen eines Koeffizienten eines Polynoms umfasst, das einen Betrag der sphärischen Aberration der Intraokularlinse umfasst, wobei das Polynom dazu geeignet ist, die Wellenfront-Aberrationsdaten des gesamten Auges zu approximieren.

**3.** Ophthalmologisches Messprogramm nach Anspruch 1 oder 2,
wobei der post-operative Berechnungsschritt umfasst: ein Berechnen der post-operativen, sphärischen Brechkraft, der post-operativen, astigmatischen Brechkraft und des post-operativen axialen Astigmatismuswinkels des gesamten Auges auf der Grundlage einer geschätzten, post-operativen, äquivalenten sphärischen Brechkraft, die durch die vorbestimmte Intraokularlinsenformel berechnet wird, die dazu geeignet ist, die post-operative Brechkraft zu berechnen.

**4.** Ophthalmologisches Messprogramm nach einem der Ansprüche 1 bis 3, wobei die Intraokularlinse eine torische Intraokularlinse umfasst, und
wobei der post-operative Berechnungsschritt ein Berechnen der post-operativen, sphärischen Brechkraft, der post-operativen, astigmatischen Brechkraft und des post-operativen, axialen Astigmatismuswinkels des gesamten Auges unter der Annahme umfasst, dass die torische Intraokularlinse vorgesehen ist, um den Hornhautastigmatismus zu beseitigen.

**5.** Ophthalmologisches Messprogramm nach einem der Ansprüche 1 bis 4, wobei der post-operative Berechnungsschritt ein Berechnen der post-operativen, sphärische Brechkraft, der post-operativen, astigmatischen Brechkraft und des post-operativen, axialen Astigmatismuswinkels des gesamten Auges unter der Bedingung, dass eine post-operative Brechkraft des gesamten Auges auf der Grundlage einer kombinierten Brechkraft aus einer Hornhautbrechkraft des Auges der zu untersuchenden Person und einer Brechkraft der in das Auge der zu untersuchenden Person zu implantierenden Intraokularlinse berechnet wird, umfasst und eine Augachsenlänge des Auges der zu untersuchenden Person der geschätzten, post-operativen Brechkraft des Auges der zu untersuchenden Person, die durch die vorbestimmte Intraokularlinsenformel berechnet wird, entspricht.

**6.** Ophthalmologisches Messprogramm nach einem der Ansprüche 1 bis 4, wobei der post-operative Berechnungsschritt umfasst
ein Kombinieren einer Hornhautbrechkraft des Auges der zu untersuchenden Person und einer Brechkraft der Intraokularlinse, die in einer Tiefe ACD einer geschätzten post-operativen, vorderen Augenkammer bezüglich der Hornhaut angeordnet ist, und
ein Berechnen einer post-operativen, sphärischen Brechkraft, der post-operativen, astigmatischen Brechkraft und des post-operativen axialen Astigmatismuswinkels des gesamten Auges unter der Annahme, dass die Retina, die sich an einer Position der Augachsenlänge des Auges der zu untersuchenden Person befindet, eine bilderzeugende Position ist.

**7.** Ophthalmologisches Messprogramm nach einem der Ansprüche 1 bis 6, wobei der post-operative Berechnungsschritt umfasst
ein Verändern einer Tiefe ACD einer geschätzten, post-operativen, vorderen Augenkammer, die eine Formel zum Gewinnen der post-operativen, sphärischen Brechkraft, der post-operativen, astigmatischen Brechkraft und des post-operativen, axialen Astigmatismuswinkels des gesamten Auges enthält, und
ein Berechnen der post-operativen, sphärischen Brechkraft, der post-operativen, astigmatischen Brechkraft, und des post-operativen, axialen Astigmatismuswinkels des gesamten Auges unter Verwendung der Tiefe ACD der geschätzten, post-operativen, vorderen Augenkammer, bei der die geschätzte, post-operative Brechkraft, die durch die vorbestimmte Intraokularlinsenformel berechnet wird, der post-operativen, sphärischen Brechkraft des gesamten Auges entspricht.

**8.** Ophthalmologisches Messprogramm nach einem der Ansprüche 1 bis 6, wobei der post-operative Berechnungsschritt umfasst
ein Verändern einer Tiefe ACD einer geschätzten, post-operativen, vorderen Augenkammer, die eine Formel zum Gewinnen der post-operativen, sphärischen Brechkraft, der post-operativen, astigmatischen Brechkraft und des post-operativen, axialen Astigmatismuswinkels des gesamten Auges enthält, und
ein Berechnen der post-operativen, sphärischen Brechkraft, der post-operativen, astigmatischen Brechkraft und des post-operativen, axialen Astigmatismuswinkels des gesamten Auges unter Verwendung der Tiefe ACD der geschätzten, post-operativen, vorderen Augenkammer, bei der eine geschätzte, post-operative, äquivalente, sphärische Brechkraft, die durch die vorbestimmte Intraokularlinsenformel berechnet wird, einer post-operativen, äquivalenten, sphärischen Brechkraft des gesamten Auges entspricht.

**9.** Ophthalmologisches Messprogramm nach Anspruch 8,
wobei der Anzeigeschritt ein Anzeigen auf dem Monitor der Tiefe ACD der geschätzten, post-operativen, vorderen Augenkammer, bei der die geschätzte, post-operative, äquivalente, sphärische Brechkraft der post-operativen, äquivalenten, sphärischen Brechkraft des gesamten Auges entspricht, umfasst.

**10.** Ophthalmologisches Messprogramm nach einem der Ansprüche 1 bis 9, wobei die post-operative Brechkraft des gesamten Auges auf der Grundlage einer kombinierten Brechkraft aus einer Hornhautbrechkraft des Auges der zu untersuchenden Person und einer sphärischen Brechkraft der in das Auge der zu untersuchenden Person zu implantierenden Intraokularlinse und einer Augachsenlänge des Auges der zu untersuchenden Person berechnet wird.

**11.** Ophthalmologisches Messprogramm nach einem der Ansprüche 1 bis 10, das ferner einen Schritt zum Berechnen der geschätzten, post-operativen Brechkraft des Auges der zu untersuchenden Person durch Einsetzen von Parametern bezüglich einer Hornhautform des Auges der zu untersuchenden Person, einer Augachsenlänge des Auges der zu untersuchenden Person und einer in das Auge der zu untersuchenden Person zu implantierenden Intraokularlinse in die vorbestimmte Intraokularlinsenformel umfasst.

**12.** Ophthalmologisches Messprogramm nach einem der Ansprüche 1 bis 11, wobei die vorbestimmte Intraokularlinsenformel eine von der SRK-Formel, der SRK II-Formel, der BINKHORST-Formel, der HOFFER-Q-Formel, der HOLLADAY-Formel oder der HAIGIS-Formel umfasst.

**13.** Ophthalmologische Simulationseinrichtung, die vorgesehen ist, um ein Simulationsbild (141) eines Retinabilds eines Auges einer zu untersuchenden Person auszugeben, wenn eine Intraokularlinse in dem Auge implantiert wird, und die umfasst ein Aberrationsdaten-Gewinnungsmittel, das dazu geeignet ist, Wellenfront-Aberrationsdaten der Hornhaut des Auges der zu untersuchenden Person zu gewinnen, die unter Verwendung einer Hornhautform-Messeinrichtung (10) gemessen wird, die vorgesehen ist, um eine Hornhautform des Auges durch Projizieren eines Placidoringziels auf die Hornhaut des Auges und Empfangen eines Reflexionslichts des Placidoringziels oder durch Verwenden optischer Interferenz zu messen,
ein Bilderzeugungsmittel zum Erzeugen eines Simulationsbilds (141) auf der Grundlage der Wellenfront-Aberrationsdaten, und
ein Anzeigesteuerungsmittel, das dazu geeignet ist, das erzeugte Simulationsbild auf einem Monitor anzuzeigen,
**dadurch gekennzeichnet, dass**:

(a) die ophthalmologische Simulationseinrichtung ein post-operatives Berechnungsmittel umfasst, das dazu geeignet ist, eine post-operative, sphärische Brechkraft, eine post-operative, astigmatische Brechkraft und einen post-operativen, axialen Astigmatismuswinkels des gesamten Auges auf der Grundlage einer geschätzten, post-operativen Brechkraft des Auges der zu untersuchenden Person, die durch eine vorbestimmte Intraokularlinsenformel berechnet wird, zu berechnen, und
(b) das Bilderzeugungsmittel dazu geeignet ist (b1) Koeffizienten eines Polynom, das die post-operative, sphärische Brechkraft, die post-operative, astigmatische Brechkraft und den post-operativen, axiale, Astigmatismuswinkel, die in dem post-operativen Berechnungsmittel berechnet werden, umfasst, und die Hornhautwellenfrontaberration dritter und höherer Ordnung, die durch das Aberrationsdaten-Gewinnungsmittel berechnet wird, zu berechnen, wobei das Polynom dazu geeignet ist, die Wellenfront-Aberrationsdaten des gesamten Auges zu approximieren, (b2) eine inverse Operation an den Wellenfront-Aberrationsdaten des gesamten Auges unter Verwendung der berechneten Koeffizienten des Polynoms auszuführen und (b3) das Simulationsbild (141) auf der Grundlage der Wellenfront-Aberrationsdaten, auf die die inverse Operation ausgeführt wird, zu erzeugen.

## Revendications

**1.** Programme de mesure ophtalmique qui est effectué dans un appareil de simulation ophtalmique conçu pour produire une image de simulation (141) d'une image rétinienne de l'oeil d'un patient à un moment où une lentille intraoculaire est implantée dans l'oeil, et conçu pour permettre à l'appareil de simulation ophtalmique d'effectuer, au moyen d'un processeur inclus dans l'appareil de simulation ophtalmique, une étape d'obtention de données d'aberration consistant à obtenir des données sur une aberration de front d'onde de la cornée de l'oeil du patient qui est mesurée en utilisant un appareil de mesure de forme de cornée (10) conçu pour mesurer une forme de cornée de l'oeil en projetant une cible de type disque de Placido sur la cornée de l'oeil et en assurant la photo-réception de la lumière de réflexion de la cible de type disque de Placido ou en utilisant un principe d'interférence optique, une étape de création d'image consistant à créer l'image de simulation (141) sur la base des données d'aberration de front d'onde, et une étape d'affichage consistant à afficher l'image de simulation créée sur un moniteur,
**caractérisé en ce que** :

(a) le programme de mesure ophtalmique comprend une étape de calcul postopératoire consistant à calculer une puissance de sphère postopératoire, une puissance astigmatique postopératoire et un angle axial astigmatique postopératoire de l'ensemble de l'oeil sur la base d'une puissance réfractive postopératoire estimée de l'oeil du patient qui est calculée par une formule de lentille intraoculaire prédéterminée, et
(b) l'étape de création d'image comprend (b1) le calcul de coefficients d'un polynôme qui comprend la puissance de sphère postopératoire, la puissance astigmatique postopératoire, et l'angle axial astigmatique postopératoire qui sont calculés dans l'étape de calcul postopératoire, et une aberration d'ordre élevé d'un troisième degré ou plus de l'aberration de front d'onde cornéenne qui est obtenue dans l'étape d'obtention de données d'aberration, le polynôme étant conçu pour approcher les données d'aberration de front d'onde de l'ensemble de l'oeil, (b2) la réalisation d'une opération inverse sur les données d'aberration de front d'onde de l'ensemble de l'oeil avec l'utilisation des coefficients calculés du polynôme, et (b3) la création de l'image de simulation (141) sur la base des données d'aberration de front d'onde sur lesquelles l'opération inverse est effectuée.

**2.** Programme de mesure ophtalmique selon la revendication 1,
dans lequel l'étape de création d'image comprend en outre le calcul d'un coefficient d'un polynôme qui comprend une quantité d'aberration sphérique de la lentille intraoculaire, le polynôme étant conçu pour approcher les données d'aberration de front d'onde de l'ensemble de l'oeil.

**3.** Programme de mesure ophtalmique selon la revendication 1 ou 2,
dans lequel l'étape de calcul postopératoire comprend le calcul de la puissance de sphère postopératoire, de la puissance astigmatique postopératoire et de l'angle axial astigmatique postopératoire de l'ensemble de l'oeil sur la base d'une puissance réfractive équivalente sphérique postopératoire estimée qui est calculée par la formule de lentille intraoculaire prédéterminée conçue pour calculer la puissance réfractive postopératoire.

**4.** Programme de mesure ophtalmique selon l'une quelconque des revendications 1 à 3,
dans lequel la lentille intraoculaire comprend une lentille intraoculaire torique, et
dans lequel l'étape de calcul postopératoire comprend le calcul de la puissance de sphère postopératoire, de la puissance astigmatique postopératoire et de l'angle axial astigmatique postopératoire de l'ensemble de l'oeil en supposant que la lentille intraoculaire torique est mise en place pour annuler l'astigmatisme cornéen.

**5.** Programme de mesure ophtalmique selon l'une quelconque des revendications 1 à 4,
dans lequel l'étape de calcul postopératoire comprend le calcul de la puissance de sphère postopératoire, de la puissance astigmatique postopératoire et de l'angle axial astigmatique postopératoire de l'ensemble de l'oeil dans une condition où la puissance réfractive postopératoire de l'ensemble de l'oeil est calculée sur la base d'une puissance réfractive combinée d'une puissance réfractive cornéenne de l'oeil du patient et d'une puissance réfractive de la lentille intraoculaire devant être implantée dans l'oeil du patient, et une longueur axiale oculaire de l'oeil du patient correspond à la puissance réfractive postopératoire estimée de l'oeil du patient qui est calculée par la formule de lentille intraoculaire prédéterminée.

**6.** Programme de mesure ophtalmique selon l'une quelconque des revendications 1 à 4,
dans lequel l'étape de calcul postopératoire comprend
la combinaison d'une puissance réfractive cornéenne de l'oeil du patient, et d'une puissance réfractive de la lentille intraoculaire qui est séparée de la cornée dans l'oeil d'une profondeur ACD d'une chambre antérieure postopératoire estimée, et
le calcul de la puissance de sphère postopératoire, de la puissance astigmatique postopératoire et de l'angle axial astigmatique postopératoire de l'ensemble de l'oeil en supposant qu'une résine qui se trouve à une position de la longueur axiale oculaire de l'oeil du patient est une position de formation d'image.

**7.** Programme de mesure ophtalmique selon l'une quelconque des revendications 1 à 6,
dans lequel l'étape de calcul postopératoire comprend,
la variation d'une profondeur ACD d'une chambre antérieure postopératoire estimée que comprend une formule conçue pour obtenir la puissance de sphère postopératoire, la puissance astigmatique postopératoire et l'angle axial astigmatique postopératoire de l'ensemble de l'oeil, et
le calcul de la puissance de sphère postopératoire, de la puissance astigmatique postopératoire et de l'angle axial astigmatique postopératoire de l'ensemble de l'oeil en utilisant la profondeur ACD de la chambre antérieure postopératoire estimée à laquelle la puissance réfractive postopératoire estimée qui est calculée par la formule de lentille intraoculaire prédéterminée correspond à la puissance de sphère postopératoire de l'ensemble de l'oeil.

**8.** Programme de mesure ophtalmique selon l'une quelconque des revendications 1 à 6,
dans lequel l'étape de calcul postopératoire comprend
la variation d'une profondeur ACD d'une chambre antérieure postopératoire estimée que comprend une formule conçue pour obtenir la puissance sphérique postopératoire, la puissance astigmatique postopératoire et l'angle axial astigmatique postopératoire de l'ensemble de l'oeil, et
le calcul de la puissance de sphère postopératoire, de la puissance astigmatique postopératoire et de l'angle axial astigmatique postopératoire de l'ensemble de l'oeil en utilisant la profondeur ACD de la chambre antérieure postopératoire estimée à laquelle une puissance réfractive équivalente sphérique postopératoire estimée qui est calculée par la formule de lentille intraoculaire prédéterminée correspond à une puissance réfractive sphérique postopératoire de l'ensemble de l'oeil.

**9.** Programme de mesure ophtalmique selon la revendication 8, dans lequel l'étape d'affichage comprend l'affichage sur le moniteur de la profondeur ACD de la chambre antérieure postopératoire estimée à laquelle la puissance réfractive équivalente sphérique postopératoire estimée correspond à la puissance équivalente sphérique postopératoire de l'ensemble de l'oeil.

**10.** Programme de mesure ophtalmique selon l'une quelconque des revendications 1 à 9, dans lequel la puissance réfractive postopératoire de l'ensemble de l'oeil est calculée sur la base d'une puissance réfractive combinée d'une puissance réfractive cornéenne de l'oeil du patient et d'une puissance de sphère de la lentille intraoculaire devant être implantée dans l'oeil du patient, et d'une longueur axiale oculaire de l'oeil du patient.

**11.** Programme de mesure ophtalmique selon l'une quelconque des revendications 1 à 10, comprenant en outre une étape de calcul de la puissance réfractive postopératoire estimée de l'oeil du patient en substituant des paramètres relatifs à une forme cornéenne de l'oeil du patient, une longueur axiale oculaire de l'oeil du patient, et une lentille intraoculaire devant être implantée dans l'oeil du patient dans la formule de lentille intraoculaire prédéterminée.

**12.** Programme de mesure ophtalmique selon l'une quelconque des revendications 1 à 11, dans lequel la formule de lentille intraoculaire prédéterminée comprend l'une quelconque de la formule SRK, de la formule SRK II, de la formule BINKHORST, de la formule HOFFER-Q, de la formule HOLLADAY et de la formule HAIGIS.

**13.** Appareil de simulation ophtalmique qui est conçu pour produire une image de simulation (141) d'une image rétinienne de l'oeil d'un patient à un moment où une lentille intraoculaire est implantée dans l'oeil, et comprend
des moyens d'obtention de données d'aberration adaptés pour obtenir des données d'aberration de front d'onde de la cornée de l'oeil du patient qui est mesurée au moyen d'un appareil de mesure de forme de cornée (10) conçu pour mesurer une forme de cornée de l'oeil en projetant une cible de type disque de Placido sur la cornée de l'oeil et en assurant la photo-réception de la lumière de réflexion de la cible de type disque de Placido ou en utilisant un principe d'interférence optique,
des moyens de création d'image adaptés pour créer l'image de simulation (141) sur la base des données d'aberration de front d'onde, et
des moyens de commande d'affichage adaptés pour afficher l'image de simulation créée sur un moniteur,
**caractérisé en ce que** :

(a) l'appareil de simulation ophtalmique comprend des moyens de calcul postopératoires adaptés pour calculer une puissance de sphère post-opératoire, une puissance astigmatique postopératoire et un angle axial astigmatique postopératoire de l'ensemble de l'oeil sur la base d'une puissance réfractive postopératoire estimée de l'oeil du patient qui est calculée par une formule de lentille intraoculaire prédéterminée, et
(b) les moyens de création d'image sont conçus pour (b1) calculer les coefficients d'un polynôme qui comprend la puissance de sphère postopératoire, la puissance astigmatique postopératoire, et l'angle axial astigmatique postopératoire qui sont calculés par les moyens de calcul postopératoire, et une aberration d'ordre élevé d'un troisième degré ou plus de l'aberration de front d'onde cornéenne qui est obtenue dans les moyens d'obtention de données d'aberration, le polynôme étant conçu pour approcher les données d'aberration de front d'onde de l'ensemble de l'oeil, (b2) réaliser une opération inverse sur les données d'aberration de front d'onde de l'ensemble de l'oeil au moyen des coefficients calculés du polynôme, et (b3) créer l'image de simulation (141) sur la base des données d'aberration de front d'onde sur lesquelles l'opération inverse est réalisée.

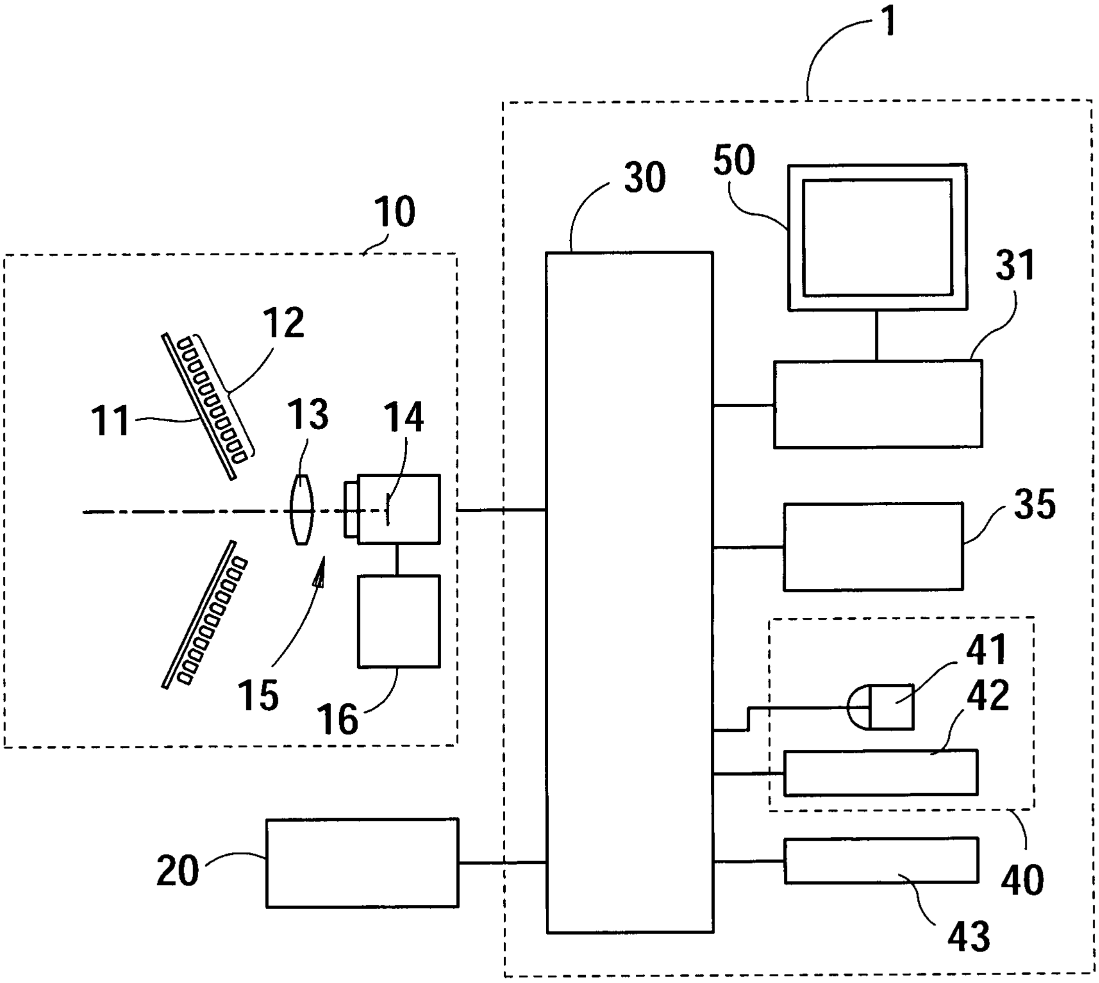


FIG.1

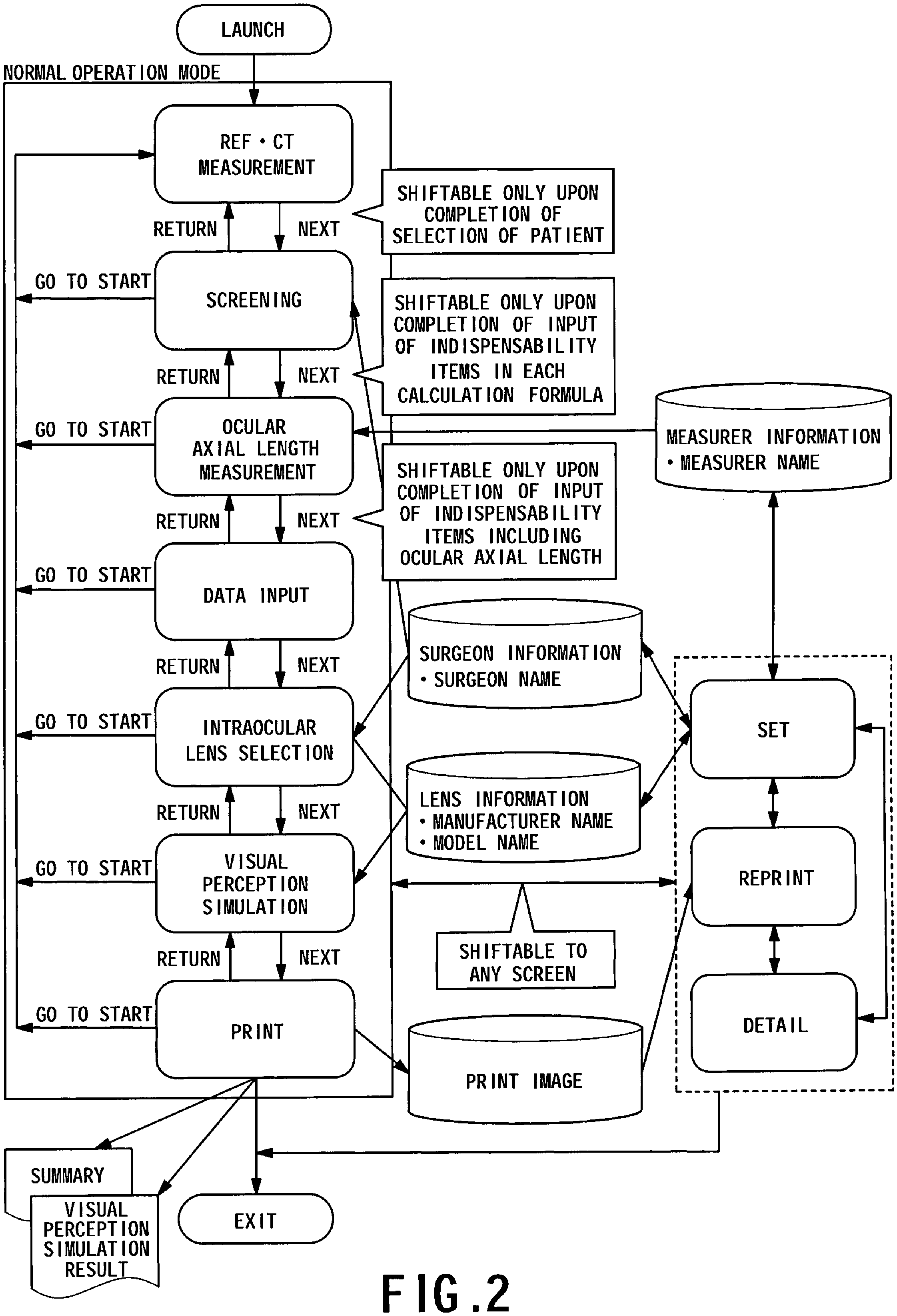
LAUNCH
NORMAL OPERATION MODE
REF • CT MEASUREMENT
RETURN
NEXT
SHIFTABLE ONLY UPON COMPLETION OF SELECTION OF PATIENT
GO TO START
SCREENING
SHIFTABLE ONLY UPON COMPLETION OF INPUT OF INDISPENSABILITY ITEMS IN EACH CALCULATION FORMULA
OCULAR AXIAL LENGTH MEASUREMENT
MEASURER INFORMATION
• MEASURER NAME
SHIFTABLE ONLY UPON COMPLETION OF INPUT OF INDISPENSABILITY ITEMS INCLUDING OCULAR AXIAL LENGTH
DATA INPUT
SURGEON INFORMATION
• SURGEON NAME
INTRAOCULAR LENS SELECTION
SET
LENS INFORMATION
• MANUFACTURER NAME
• MODEL NAME
VISUAL PERCEPTION SIMULATION
REPRINT
SHIFTABLE TO ANY SCREEN
DETAIL
PRINT
PRINT IMAGE
SUMMARY
VISUAL PERCEPTION SIMULATION RESULT
EXIT

FIG.2

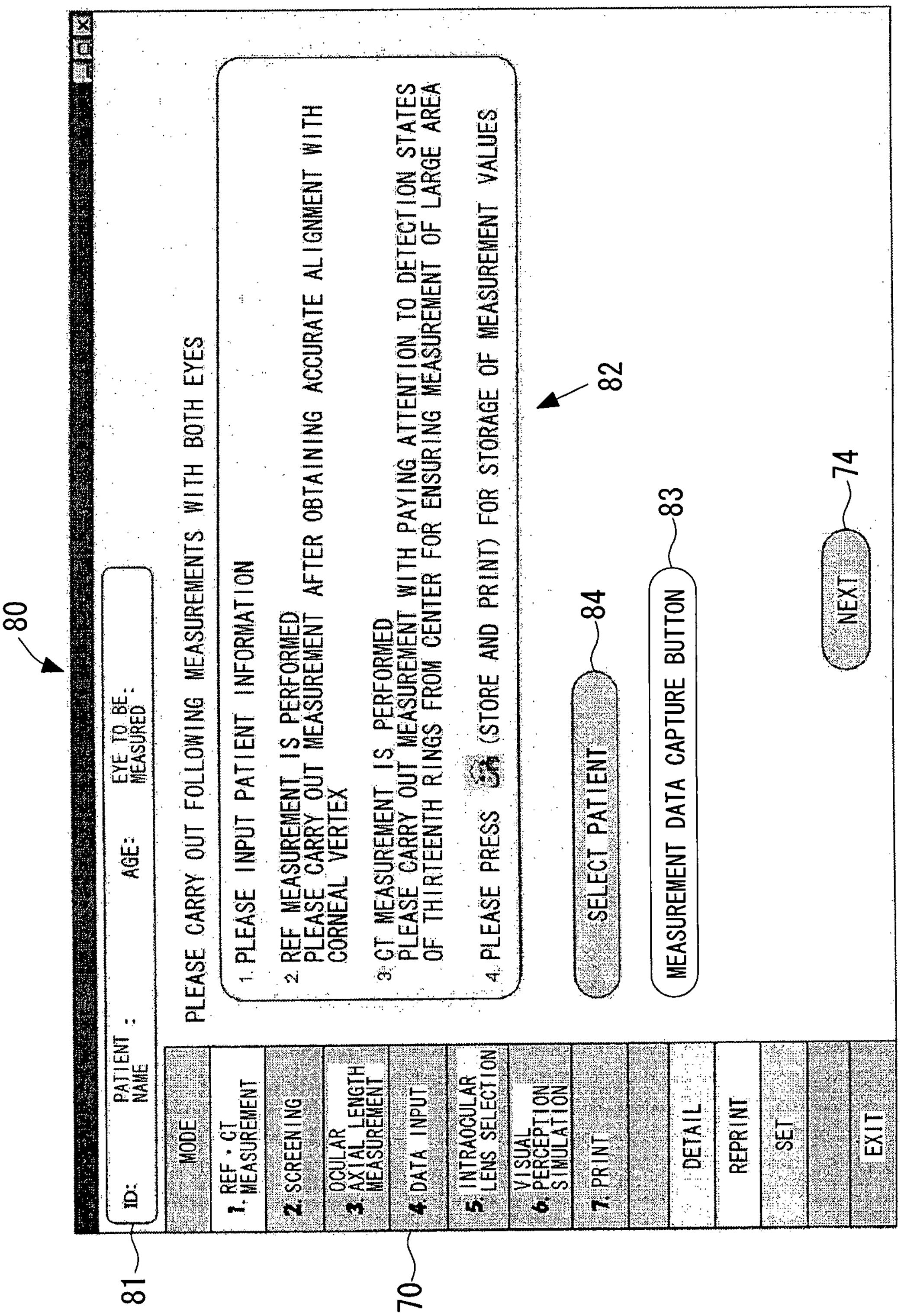
80
81
70
82
83
84
74
ID:
PATIENT NAME :
AGE:
EYE TO BE MEASURED :
MODE
1. REF · CT MEASUREMENT
2. SCREENING
3. OCULAR AXIAL LENGTH MEASUREMENT
4. DATA INPUT
5. INTRAOCULAR LENS SELECTION
6. VISUAL PERCEPTION SIMULATION
7. PRINT
DETAIL
REPRINT
SET
EXIT
PLEASE CARRY OUT FOLLOWING MEASUREMENTS WITH BOTH EYES
1. PLEASE INPUT PATIENT INFORMATION
2. REF MEASUREMENT IS PERFORMED
PLEASE CARRY OUT MEASUREMENT AFTER OBTAINING ACCURATE ALIGNMENT WITH CORNEAL VERTEX
3. CT MEASUREMENT IS PERFORMED
PLEASE CARRY OUT MEASUREMENT WITH PAYING ATTENTION TO DETECTION STATES OF THIRTEENTH RINGS FROM CENTER FOR ENSURING MEASUREMENT OF LARGE AREA
4. PLEASE PRESS (STORE AND PRINT) FOR STORAGE OF MEASUREMENT VALUES
SELECT PATIENT
MEASUREMENT DATA CAPTURE BUTTON
NEXT

FIG. 3

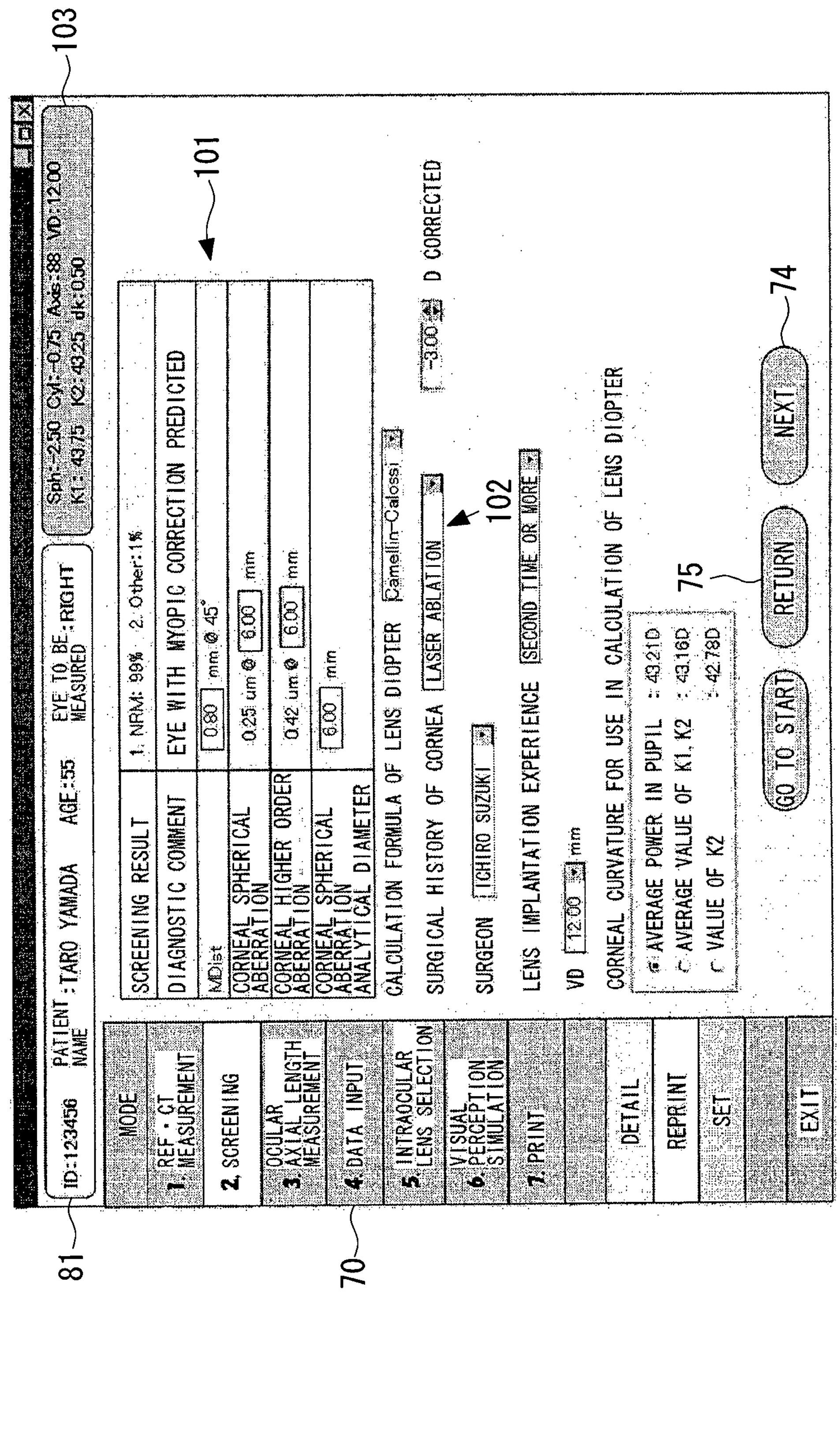
100
103
101
102
74
75
81
70
ID:123456
PATIENT NAME :TARO YAMADA
AGE:55
EYE TO BE MEASURED :RIGHT
Sph:-2.50 Cyl:-0.75 Axs:88 VD:12.00
K1: 43.75 K2: 43.25 dk:0.50
MODE
1. REF · CT MEASUREMENT
2. SCREENING
3. OCULAR AXIAL LENGTH MEASUREMENT
4. DATA INPUT
5. INTRAOCULAR LENS SELECTION
6. VISUAL PERCEPTION SIMULATION
7. PRINT
DETAIL
REPRINT
SET
EXIT
SCREENING RESULT
1. NRM: 99% 2. Other: 1%
DIAGNOSTIC COMMENT
EYE WITH MYOPIC CORRECTION PREDICTED
MDist
0.80 mm @ 45°
CORNEAL SPHERICAL ABERRATION
0.25 um @ 6.00 mm
CORNEAL HIGHER ORDER ABERRATION
0.42 um @ 6.00 mm
CORNEAL SPHERICAL ABERRATION ANALYTICAL DIAMETER
6.00 mm
CALCULATION FORMULA OF LENS DIOPTER
Camellin-Calossi
SURGICAL HISTORY OF CORNEA
LASER ABLATION
-3.00 D CORRECTED
SURGEON
ICHIRO SUZUKI
LENS IMPLANTATION EXPERIENCE
SECOND TIME OR MORE
VD
12.00 mm
CORNEAL CURVATURE FOR USE IN CALCULATION OF LENS DIOPTER
AVERAGE POWER IN PUPIL : 43.21D
AVERAGE VALUE OF K1, K2 : 43.16D
VALUE OF K2 : 42.78D
GO TO START
RETURN
NEXT

FIG. 4

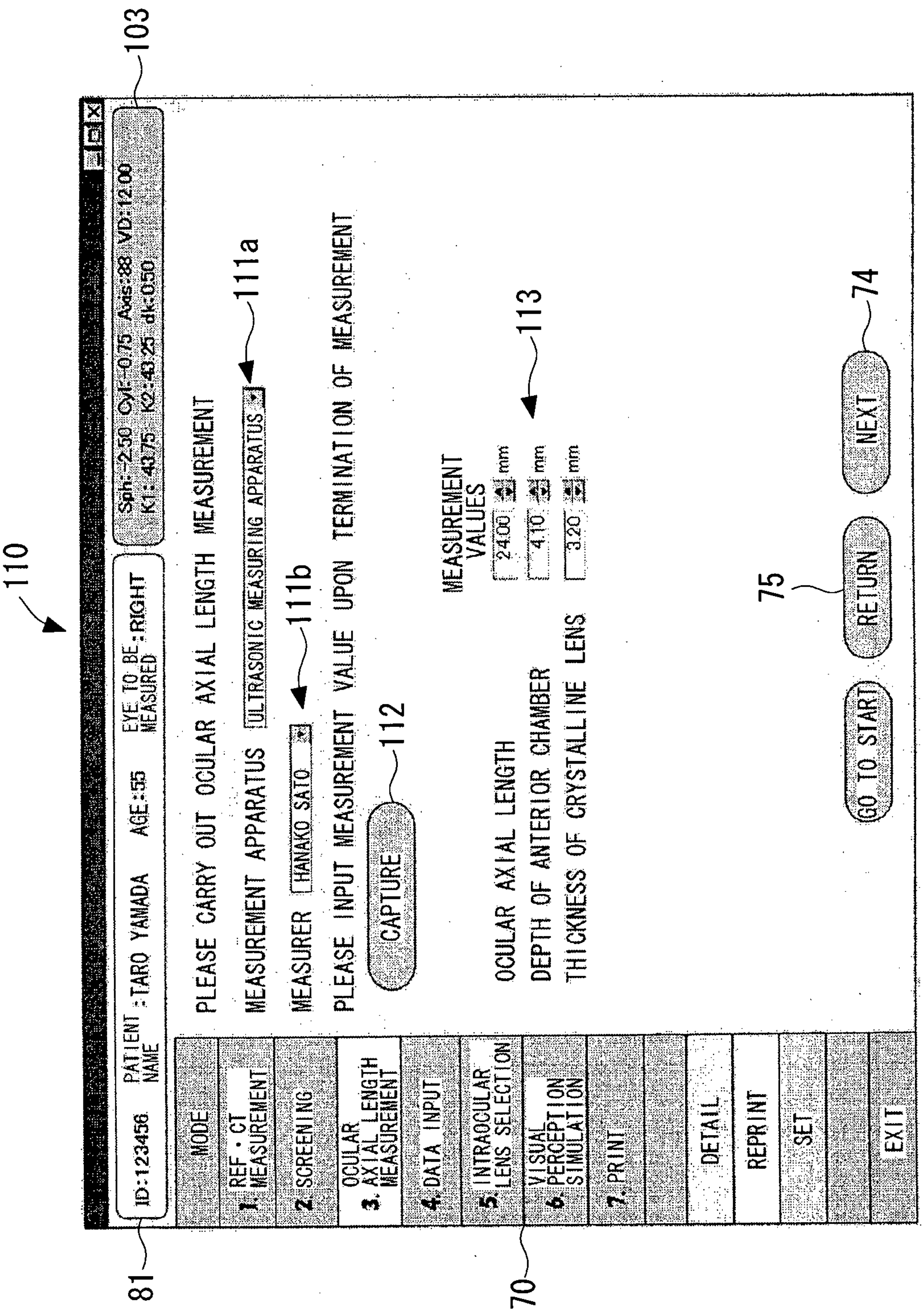
110
103
81
70
ID:123456
PATIENT NAME :TARO YAMADA
AGE:55
EYE TO BE MEASURED :RIGHT
Sph:-2.50 Cyl:-0.75 Axis:88 VD:12.00
K1:43.75 K2:43.25 dk:0.50
MODE
1. REF・CT MEASUREMENT
2. SCREENING
3. OCULAR AXIAL LENGTH MEASUREMENT
4. DATA INPUT
5. INTRAOCULAR LENS SELECTION
6. VISUAL PERCEPTION SIMULATION
7. PRINT
DETAIL
REPRINT
SET
EXIT
PLEASE CARRY OUT OCULAR AXIAL LENGTH MEASUREMENT
MEASUREMENT APPARATUS
ULTRASONIC MEASURING APPARATUS
111a
MEASURER
HANAKO SATO
111b
PLEASE INPUT MEASUREMENT VALUE UPON TERMINATION OF MEASUREMENT
CAPTURE
112
MEASUREMENT VALUES
113
OCULAR AXIAL LENGTH
24.00 mm
DEPTH OF ANTERIOR CHAMBER
4.10 mm
THICKNESS OF CRYSTALLINE LENS
3.20 mm
GO TO START
RETURN
75
NEXT
74

FIG. 5

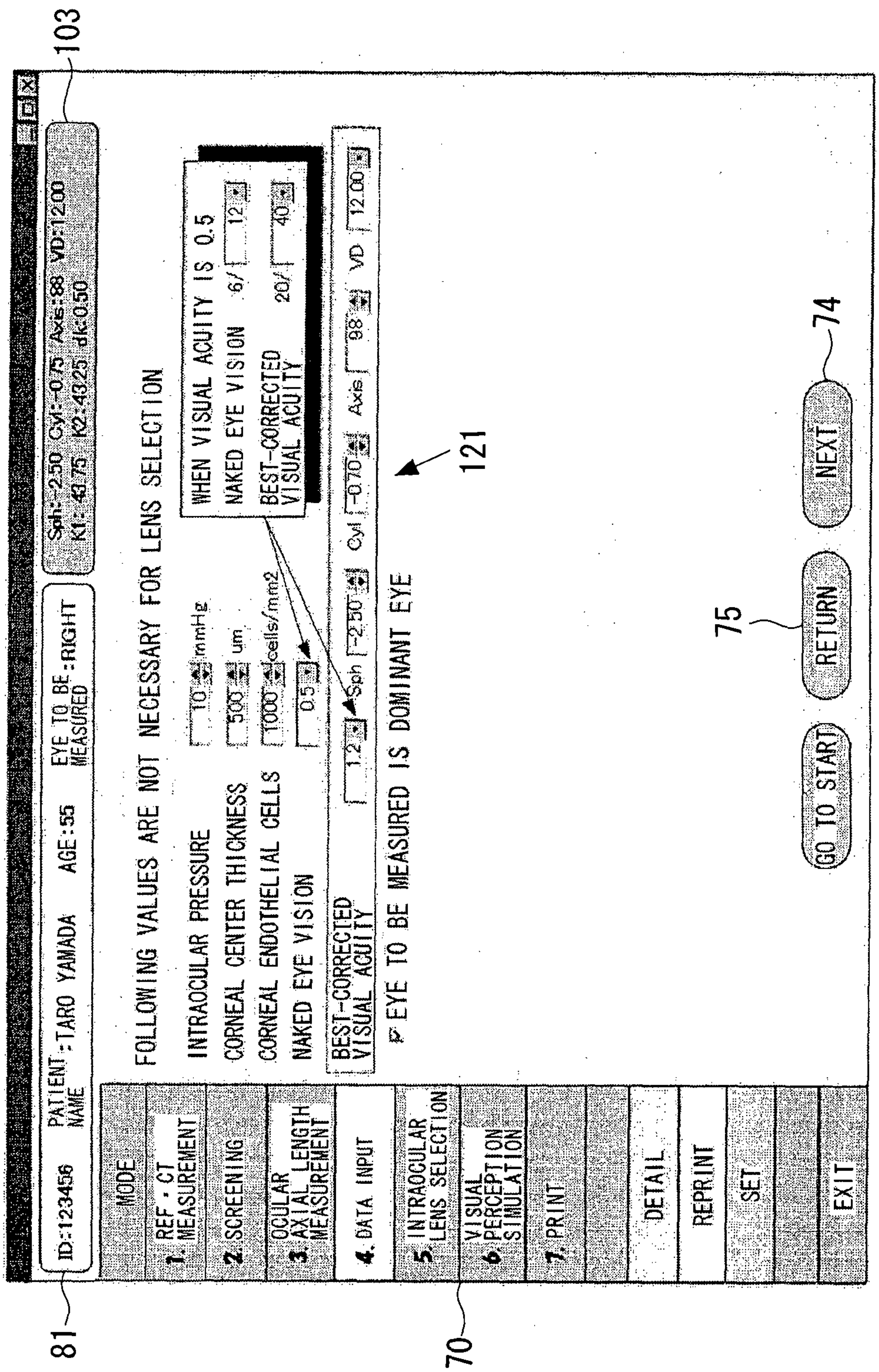
120
103
81
70
121
75
74
ID:123456
PATIENT NAME :TARO YAMADA
AGE:55
EYE TO BE MEASURED :RIGHT
Sph:-2.50 Cyl:-0.75 Axis:88 VD:12.00
K1:43.75 K2:43.25 dk:0.50
MODE
1. REF・CT MEASUREMENT
2. SCREENING
3. OCULAR AXIAL LENGTH MEASUREMENT
4. DATA INPUT
5. INTRAOCULAR LENS SELECTION
6. VISUAL PERCEPTION SIMULATION
7. PRINT
DETAIL
REPRINT
SET
EXIT
FOLLOWING VALUES ARE NOT NECESSARY FOR LENS SELECTION
INTRAOCULAR PRESSURE 10 mmHg
CORNEAL CENTER THICKNESS 500 um
CORNEAL ENDOTHELIAL CELLS 1000 cells/mm2
NAKED EYE VISION 0.5
BEST-CORRECTED VISUAL ACUITY 1.2 Sph -2.50 Cyl -0.70 Axis 98 VD 12.00
EYE TO BE MEASURED IS DOMINANT EYE
WHEN VISUAL ACUITY IS 0.5
NAKED EYE VISION 6/ 12
BEST-CORRECTED VISUAL ACUITY 20/ 40
GO TO START
RETURN
NEXT

FIG. 6

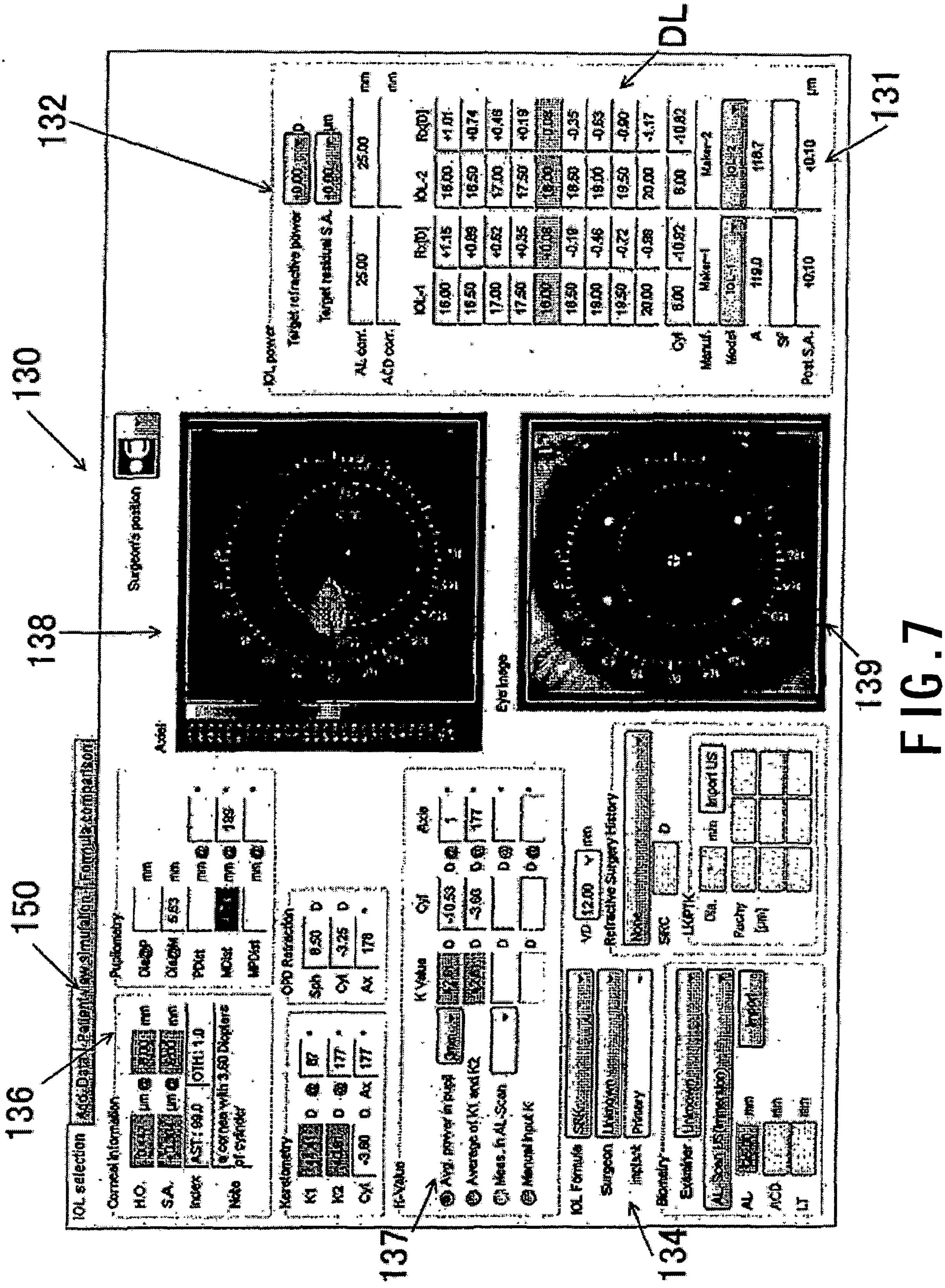
130
131
132
134
136
137
138
139
150
DL
IOL selection
Surgeon's position
Eye Image
IOL power
Target refractive power
Target residual S.A.
Corneal information
Keratometry
Pupillometry
OPD Refraction
K-Value
IOL Formula
Biometry
Refractive Surgery History

FIG.7

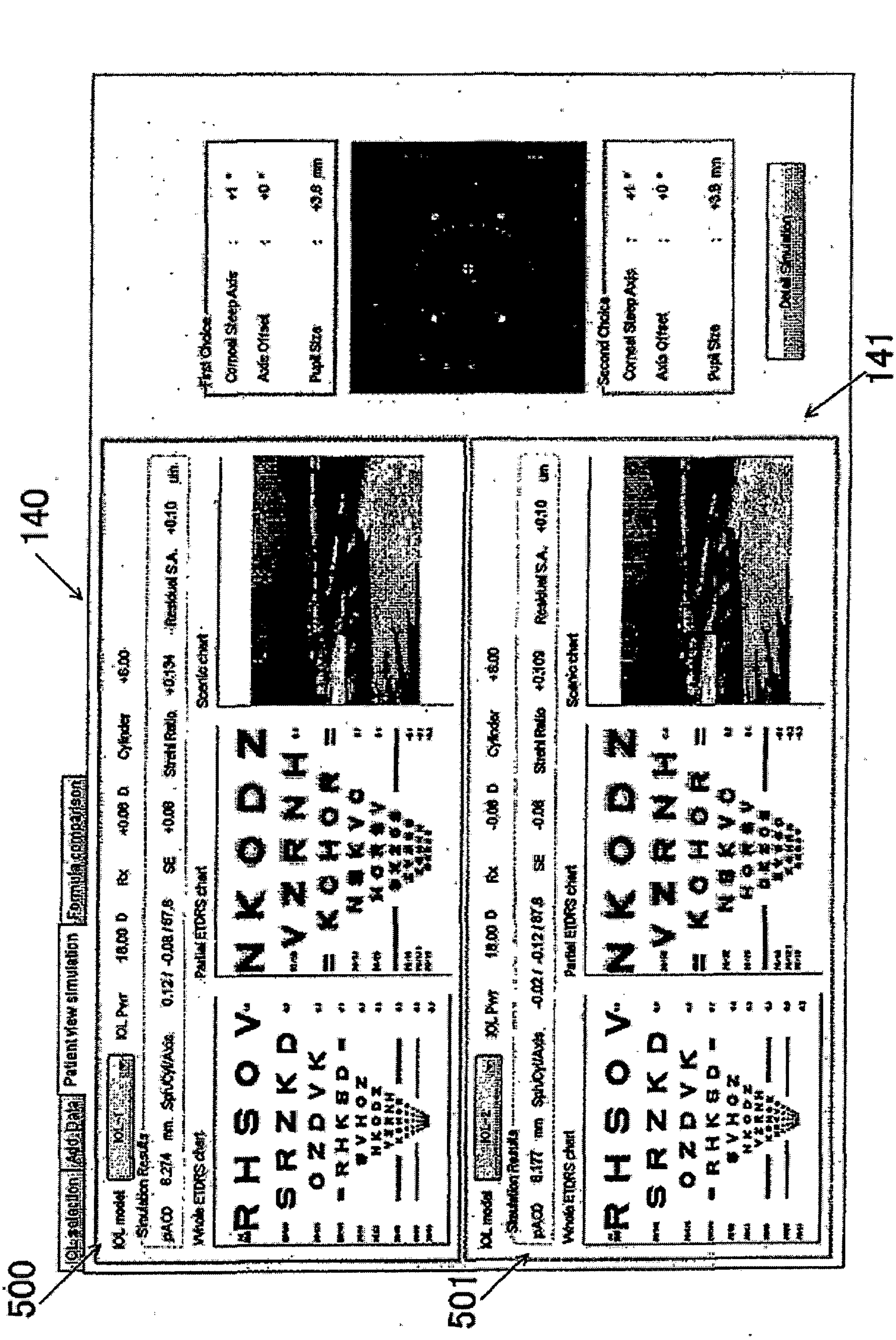
140
141
500
501
IOL selection
Add Data
Patient view simulation
Formula comparison
IOL model
IOL Pwr
18.00 D
Rx
+0.08 D
-0.08 D
Cylinder
+6.00
Simulation Results
pACD
6.274
6.177
mm
Sph/Cyl/Axis
0.12 / -0.08 / 87.8
-0.02 / -0.12 / 87.8
SE
+0.08
-0.08
Strehl Ratio
+0.134
+0.109
Residual S.A.
+0.10
um
Whole ETDRS chart
Partial ETDRS chart
Scenic chart
First Choice
Second Choice
Corneal Steep Axis
Axis Offset
Pupil Size
+3.8 mm
Detail Simulation

FIG.8

(1)Denormalized IOL refractive power (IOL)

$$IOL=A-2.6\times AL-0.9\times K-DR\times(0.0875\times A-8.55)$$

(2)Post-operative refractive power (ERROR)

$$ERROR=(A-2.5\times AL-0.9\times K-LP)/(0.0875\times A-8.55)$$

K:Corneal refractive power [D] $K=(n_k-1.000)\times 1000/R^{*1}$

AL:Ocular axial length [mm]

A:A constant

DR:Refractive power [D] of a corrective lens that is desired after operation (+value: hyperopia, -value: myopia )

LP:Refractive power [D] of an IOL to be implanted

FIG.9

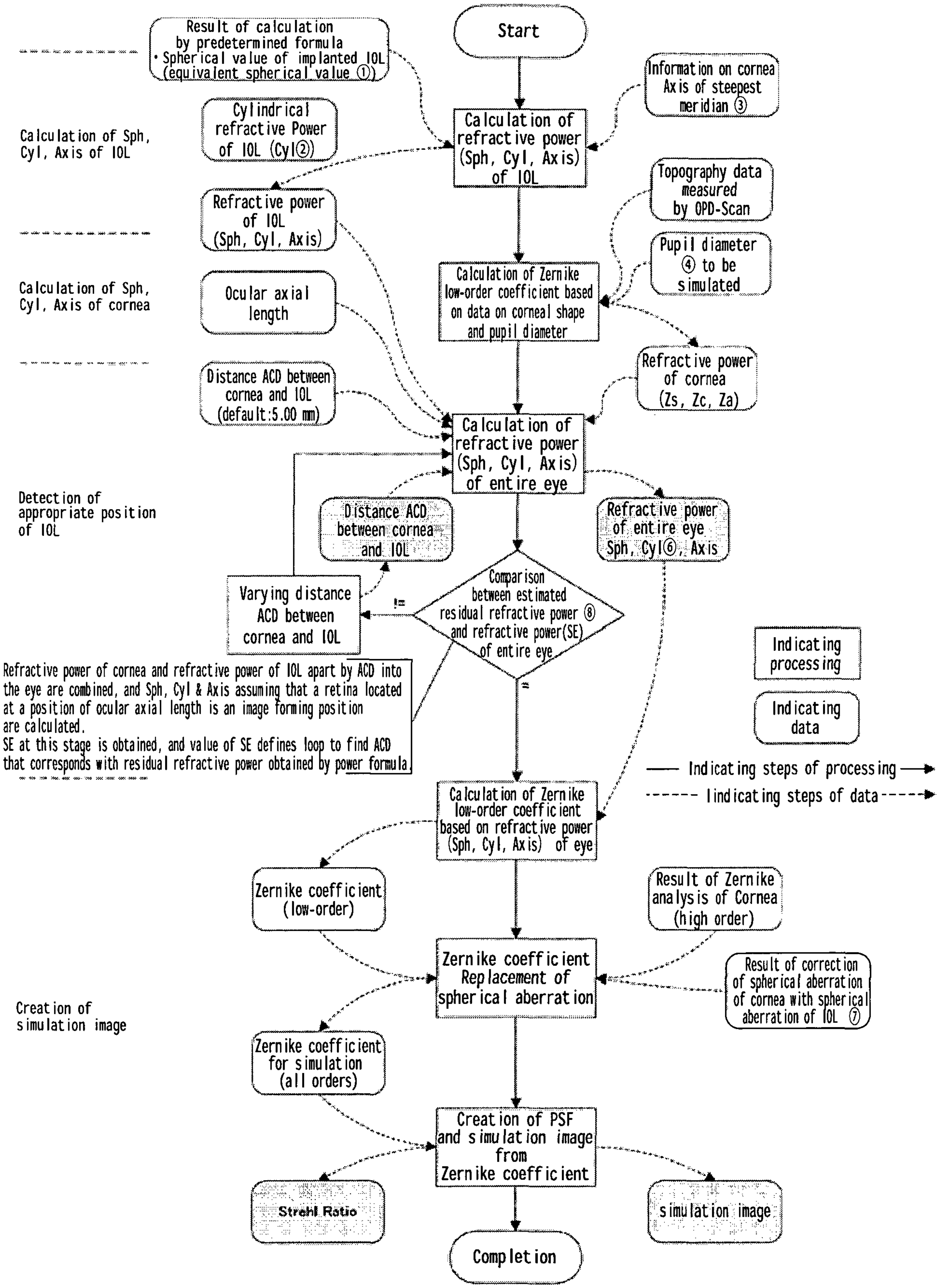
Result of calculation by predetermined formula
• Spherical value of implanted IOL (equivalent spherical value ①)
Start
Information on cornea Axis of steepest meridian ③
Calculation of Sph, Cyl, Axis of IOL
Cylindrical refractive Power of IOL (Cyl②)
Calculation of refractive power (Sph, Cyl, Axis) of IOL
Refractive power of IOL (Sph, Cyl, Axis)
Topography data measured by OPD-Scan
Pupil diameter ④ to be simulated
Calculation of Sph, Cyl, Axis of cornea
Ocular axial length
Calculation of Zernike low-order coefficient based on data on corneal shape and pupil diameter
Refractive power of cornea (Zs, Zc, Za)
Distance ACD between cornea and IOL (default:5.00 mm)
Calculation of refractive power (Sph, Cyl, Axis) of entire eye
Detection of appropriate position of IOL
Distance ACD between cornea and IOL
Refractive power of entire eye Sph, Cyl⑥, Axis
Varying distance ACD between cornea and IOL
!=
Comparison between estimated residual refractive power ⑧ and refractive power(SE) of entire eye
=
Indicating processing
Indicating data
Refractive power of cornea and refractive power of IOL apart by ACD into the eye are combined, and Sph, Cyl & Axis assuming that a retina located at a position of ocular axial length is an image forming position are calculated.
SE at this stage is obtained, and value of SE defines loop to find ACD that corresponds with residual refractive power obtained by power formula
Indicating steps of processing
Iindicating steps of data
Calculation of Zernike low-order coefficient based on refractive power (Sph, Cyl, Axis) of eye
Zernike coefficient (low-order)
Result of Zernike analysis of Cornea (high order)
Zernike coefficient Replacement of spherical aberration
Result of correction of spherical aberration of cornea with spherical aberration of IOL ⑦
Creation of simulation image
Zernike coefficient for simulation (all orders)
Creation of PSF and simulation image from Zernike coefficient
Strehl Ratio
simulation image
Completion

FIG.10

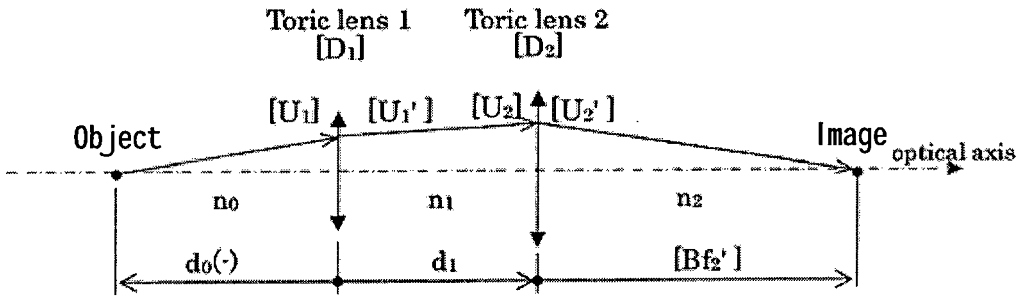
Toric lens 1
[D1]
Toric lens 2
[D2]
[U1]
[U1']
[U2]
[U2']
Object
Image
optical axis
n0
n1
n2
d0(-)
d1
[Bf2']

FIG.11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2009281552 A1
- JP 2009034451 A
- JP HEI10108837 B
- JP 2005288176 A
- JP 2009034441 A